# EUROPEAN PATENT SPECIFICATION

(11) **EP 3 693 875 B1**
(45) Date of publication and mention of the grant of the patent: **29.11.2023**
(21) Application number: 18864756.4
(22) Date of filing: 02.10.2018
(51) Int. Cl.: G06F 21/32, G01N 23/18, G06T 7/00, H04L 9/40, G06F 21/31, G06F 21/35, G06F 21/60, G06F 21/62

(54) **FOOD PRODUCT PROCESSING DEVICE, FOOD PRODUCT PROCESSING DEVICE MANAGEMENT SYSTEM, AND FOOD PRODUCT PROCESSING DEVICE MANAGEMENT METHOD**
VORRICHTUNG ZUR VERARBEITUNG VON NAHRUNGSMITTELN, SYSTEM ZUR VERWALTUNG EINER VORRICHTUNG ZUR VERARBEITUNG VON NAHRUNGSMITTELN UND VERFAHREN ZUR VERWALTUNG EINER VORRICHTUNG ZUR VERARBEITUNG VON NAHRUNGSMITTELN
DISPOSITIF DE TRAITEMENT DE PRODUIT ALIMENTAIRE, SYSTÈME DE GESTION DE DISPOSITIF DE TRAITEMENT DE PRODUIT ALIMENTAIRE ET PROCÉDÉ DE GESTION DE DISPOSITIF DE TRAITEMENT DE PRODUIT ALIMENTAIRE

(30) Priority: 02.10.2017 JP 2017193053; 02.10.2017 JP 2017193054; 02.10.2017 JP 2017193055
(43) Date of publication of application: 12.08.2020
(73) Proprietor: Ishida Co., Ltd., Kyoto-shi, Kyoto 606-8392 (JP)
(72) Inventor: TSUGAWA, Tono, Kyoto-shi Kyoto 606-8392 (JP); HATTA, Shigetoshi, Ritto-shi Shiga 520-3026 (JP); TAKEMURA, Yukihiro, Tokyo 173-0004 (JP); TAKAMORI, Kazuaki, Ritto-shi Shiga 520-3026 (JP); YAMADA, Yuzuru, Ritto-shi Shiga 520-3026 (JP); HAYAMIZU, Masahiro, Ritto-shi Shiga 520-3026 (JP)
(74) Representative: Global IP Europe Patentanwaltskanzlei
(86) International application number: PCT/JP2018/036945
(87) International publication number: WO 2019/069952

(56) References cited:
- EP-A2- 1 621 944
- EP-B1- 1 621 944
- WO-A1-2013/012798
- WO-A1-2017/056986
- JP-A- 2005 192 843
- JP-A- 2007 004 630
- JP-A- 2007 280 216
- JP-A- 2008 296 201
- JP-A- 2008 510 216
- JP-A- 2010 061 397
- JP-A- 2012 243 046
- JP-A- 2014 071 072
- JP-A- 2016 134 137
- US-A1- 2008 109 895

## Description

### TECHNICAL FIELD

The present invention relates to a food-processing device, a food-processing device management system, and a food-processing device management method.

### BACKGROUND ART

Various food-processing devices are used in steps for manufacturing food. Examples of food-processing devices include, *inter alia,* a device that inspects for foreign substances in food using X-rays and light (visible light, infrared rays, ultraviolet rays, etc.), a device that measures food weight and sorts the food, a device that checks a seal part of food packaging, a device that performs printing on a label attached to food packaging, and a device that manages food temperature, concentrations of medicine used in the food, etc. With such food-processing devices, adjustment and testing of detection sensitivity, etc., must be appropriately performed before the device is used, in order to guarantee food safety and quality. However, adjustment and testing of detection sensitivity, etc., cannot be appropriately performed except by a knowledgeable and skilled manager who has received prescribed training. Additionally, confirmation of the content printed on the label, regular maintenance management of the food-processing device, etc., must also be performed by an authorized and responsible manager. Therefore, when dealing with specific processes that should be performed by a manager who is a user having prescribed authority, food-processing devices conventionally have a function of determining whether or not a user is a manager and performing user authentication when the user is a manager.

For example, as disclosed in JP 2014-71072 A), there is a known food-processing device which has an antenna and which, when a user holding a prescribed terminal approaches the antenna, communicates information with the terminal held by the user through the user's body to perform a user authentication process. Additionally, the user authentication process conventionally involves utilizing ID cards assigned to each user, passwords set for each user, etc.

EP 1 621 944 A2 discloses a food-processing device configured to process a food in a line that manufactures the food, the food-processing device comprising:
an imaging device configured to capture an image of an eye of a user of the food-processing device;
an authentication part configured to authenticate the user on the basis of the image of the user's eye captured by the imaging device; and
a management part configured to make a process performed by the processor executable for the user authenticated by the authentication part.

### SUMMARY OF THE INVENTION

### <Technical Problem>

However, when a user authentication process of a food-processing device utilizes terminals or ID cards held by the users or passwords set for each user, the users sometimes share or lend and borrow terminals, ID cards, or passwords. In this case, there is a concern of a user being authenticated who should not be, and of the user being able to execute processes requiring prescribed authority. There is also a concern that a password for authentication will be widely known among users other than the proper users of the password, or that an ID card for authentication will be lent and borrowed among a plurality of users, thereby leading to situations in which an authenticated user is not the same person as an actual user.

The present invention was devised in order to solve the problems described above, it being an object of the invention to provide a food-processing device with which situations in which a user is authenticated who should not be and situations in which an authenticated user is not the same person as an actual user are avoided, and only an authenticated user can execute a process.

Another object of the present invention is to provide a food-processing device with which use of the device by a user other than the authenticated user can be suppressed even when the user moves away from the proximity of the food-processing device after authentication.

Another object of the present invention is to provide a food-processing device management system and a food-processing device management method with which information used in an authentication process can easily be managed when managing a plurality of food-processing devices having a function of authenticating users.

### <Solution to Problem>

The invention is defined in the independent claims 1, 16 and 25. A food-processing device according to the present invention is a device that comprises a processor, an imaging device, an authentication part, and a management part, and processes a food. The processor is configured to perform at least one process on the food in a line in which the food is manufactured. The imaging device is configured to capture an image of an eye of a user of the food-processing device. The authentication part is configured to perform authentication of the user on the basis of the image of the user's eye captured by the imaging device. The management part is configured to make the process performed by the processor executable for the user authenticated by the authentication part. The authentication part is configured to authenticate the user on the basis of information of the user's iris included in the image of the user's eye.

This food-processing device utilizes the image of the user's eye as biometric information for authenticating the user. Situations in which a user is authenticated who should not be, and situations in which the authenticated user is not the same person as the actual user are thereby avoided. Consequently, only an authenticated user can execute a process using the food-processing device. Additionally, with the food-processing device, the user can be authenticated with high precision by using an iris included in the image of the user's eye to authenticate the user.

The food-processing device according to the present invention preferably further comprises a determination part configured to determine whether or not the user is present inside a prescribed region. In this case, the management part is configured to automatically terminate the executable state of the process performed by the processor on the basis of a determination result from the determination part.

In this food-processing device, the user authentication is automatically terminated on the basis of the region in which the authenticated user is present. Therefore, with this food-processing device, use of the device by a user other than the authenticated user can be suppressed.

In the food-processing device according to the present invention, the determination part is configured to preferably determine whether or not the user is present inside the prescribed region proximal to the food-processing device. The management part configured to automatically terminate the executable state of the process performed by the processor when the determination part determines that the user is not present inside the prescribed region.

In this food-processing device, the user authentication is automatically terminated when the authenticated user is not present in the proximity of the device. Consequently, with this food-processing device, use of the device by a user other than the authenticated user can be suppressed.

In the food-processing device according to the present invention, the determination part configured to preferably sense whether or not an indicator held by the user is present inside the prescribed region to determine whether or not the user is present inside the prescribed region.

In this food-processing device, the indicator held by the user can be utilized to ascertain with high precision whether or not the user is present in the proximity of the device. Consequently, with this food-processing device, the precision of the process of automatically terminating the user authentication can be improved.

The food-processing device according to the present invention preferably further comprises a first storage part that correlates and stores the user and the indicator. The determination part is capable of sensing a plurality of indicators distinctly from each other. The determination part is configured to determine that the user is not present inside the prescribed region upon sensing that the indicator which is stored in the first storage part and which corresponds to the user authenticated by the authentication part is not present inside the prescribed region.

With this food-processing device, it is possible to determine whether or not each user holding an indicator is present in the proximity of the device. Consequently, with this food-processing device, it is possible to suppress instances in which a user other than the authenticated user uses the device before being authenticated themselves.

The food-processing device according to the present invention preferably further comprises a second storage part that correlates and stores the user and the process made executable for the user by the management part.

With this food-processing device, it is possible for an executable process to be set in advance for each user by correlating the user of the device and the process that is executable for the user and storing this correlation in advance.

In the food-processing device according to the present invention, the second storage part preferably correlates and stores: an authentication level which is one of a plurality of authentication levels and which is set for each user; and the process made executable by the management part, this process being set for each authentication level.

With this food-processing device, processes that are executable for each user can easily be managed by setting an authentication level for each user because executable processes can be set in advance for each authentication level.

In the food-processing device according to the present invention, the authentication part is preferably configured to further authenticate, on the basis of information other than an image of the user's eye, users for whom the highest authentication level among the plurality of authentication levels has been set.

With this food-processing device, the users for whom the highest authentication level has been set can be authenticated using a password, etc., rather than an image of an eye. For that reason, with this food-processing device, appropriate countermeasures can be taken even in times of emergency, such as when, for example, malfunctions occur in the eye image authentication function and the imaging device. Furthermore, a special management mode for the device can be provided.

In the food-processing device according to the present invention, the management part is preferably configured to make a first-type process performed by the processor executable for only users authenticated by the authentication part and makes a second-type process performed by the processor executable for all the users. In this case, the processor is configured to perform at least two kinds of processes including the first-type process and the second-type process on the food.

With this food-processing device, a process requiring user authentication and a process not requiring user authentication can be distinguished, and there is therefore no need to perform the authentication process for a user who will not perform a critical process requiring special authority.

The food-processing device according to the present invention preferably further comprises a third storage part that correlates and stores the user authenticated by the authentication part and the process made executable for the user by the management part.

With this food-processing device, the authenticated user and post-authentication operation specifics of the user are correlated and stored, whereby the traceability of the food can be improved.

In the food-processing device according to the present invention, the third storage part is preferably configured to not accept changes or deletions to the correlated and stored user authenticated by the authentication part and the correlated and stored process made executable for the user by the management part.

With this food-processing device, falsification of this information can be prevented and the reliability of the stored information can be improved because the already stored user and the post-authentication operation specifics of the user cannot be changed or deleted.

The food-processing device according to the present invention preferably further comprises a fourth storage part that correlates and stores the image of the user's eye captured by the imaging device and user identification information for uniquely identifying the user.

With this food-processing device, the processes of registering and referencing the image of the user's eye can be made easier by, for example, correlating and storing an ID number (user identification information) of the user and the image of the user's eye.

In the food-processing device according to the present invention, the imaging device is preferably configured such that at least one of a position and an orientation of the imaging device is adjustable in accordance with the position of the user's eye.

With this food-processing device, an image of the user's eye can easily be acquired in accordance with the height of the user.

In the food-processing device according to the present invention, it is preferable that when a prescribed time has elapsed after the user was authenticated by the authentication part, the management part renders the process performed by the processor unexecutable until the user is again authenticated by the authentication part.

With this food-processing device, another authentication of the user is required when the prescribed time has elapsed after the user has been authenticated. For that reason, this food-processing device is capable of suppressing instances in which an actual user continues to be able to execute the process when the authenticated user is not the same person as the actual user.

In the food-processing device according to the present invention, the processor is preferably configured to perform a process determining the presence/absence of foreign substances included in the food.

With this food-processing device, safety and quality of the food can be guaranteed by detecting foreign substances included in the food.

A food-processing device management system according to the present invention comprises a biometric information acquisition part, one first storage part, a second storage part, an authentication part, and a management part, the system managing a plurality of food-processing devices that process food in a line in which the food is manufactured. The biometric information acquisition part configured to acquire biometric information of users of the food-processing devices. The first storage part correlates and stores, for each user, user identification information for uniquely identifying the user; and the biometric information. The second storage part stores executability information pertaining to processes the users can execute using the food-processing devices. The authentication part is configured to authenticate the users on the basis of the biometric information acquired by the biometric information acquisition part and the biometric information stored in the first storage part. The management part, on the basis of the executability information stored in the second storage part, is configured to make the process performed by the food-processing devices executable for the users authenticated by the authentication part. The biometric information is information of the user's iris included in an image of the user's eye.

This food-processing device management system is capable of collectively managing data of the users' biometric information that is required for the process of authenticating users of the food-processing devices. Therefore, with this food-processing device management system, managing information used in the authentication process can be made easier when managing a plurality of food-processing devices having a function of authenticating users.

The food-processing device management system according to the present invention preferably further comprises a plurality of the food-processing devices.

This food-processing device management system is capable of collectively managing data of the users' biometric information that is required for the process of authenticating users of the plurality of food-processing devices. Therefore, with this food-processing device management system, managing information used in the authentication process can be made easier when managing the plurality of food-processing devices having the function of authenticating users.

In the food-processing device management system according to the present invention, the food-processing devices are preferably configured to perform the process of determining the presence/absence of foreign substances included in the food.

With this food-processing device management system, safety and quality of the food can be guaranteed by detecting foreign substances included in the food with the food-processing devices.

In the food-processing device management system according to the present invention, each of the plurality of food-processing devices preferably has at least one biometric information acquisition part. In this case, the first storage part correlates and stores the biometric information acquired by any of the biometric information acquisition parts; and the user identification information.

With this food-processing device management system, once a user registers biometric information in any one food-processing device, the user is capable of performing the authentication process on all the food-processing devices.

In the food-processing device management system according to the present invention, each of the plurality of food-processing devices preferably has the second storage part.

With this food-processing device management system, the processes executable for the users can be managed in each food-processing device.

The food-processing device management system according to the present invention preferably further comprises a third storage part that correlates and stores the user authenticated by the authentication part and the process made executable for the user by the management part.

With this food-processing device management system, the authenticated user and post-authentication operation specifics of the user are correlated and stored, whereby the traceability of the food can be improved.

In the food-processing device management system according to the present invention, the third storage part is preferably configured to not accept changes or deletions to the correlated and stored user authenticated by the authentication part and the correlated and stored process made executable for the user by the management part.

With this food-processing device management system, falsification of this information can be prevented and the reliability of the stored information can be improved because the already stored user and the post-authentication operation specifics of the user cannot be changed or deleted.

In the food-processing device management system according to the present invention, the third storage part preferably correlates and stores, for each of the food-processing devices, the user authenticated by the authentication part and the process made executable for the user by the management part.

With this food-processing device management system, the authenticated users and the post-authentication operation specifics of users can be correlated and collectively managed across the plurality of food-processing devices.

In the food-processing device management system according to the present invention, the second storage part preferably correlates and stores the user identification information and the executability information.

With this food-processing device management system, the process by which the executable processes are registered and referenced for each user can be made easier because the users of the food-processing devices and the processes executable for the users are correlated and stored.

A food-processing device management method according to the present invention is a method for managing a plurality of food-processing devices that process a food, wherein biometric information of users of the food-processing devices is firstly acquired. Next, with this method, user identification information for uniquely identifying the user; and biometric information are correlated and stored for each user. Next, with this method, executability information pertaining to a process that is executable for the user by using the food-processing device is stored. Next, with this method, the user is authenticated on the basis of the acquired biometric information and the biometric information stored in correlation with the user identification information. Next, with this method, the process performed by the food-processing device is made executable for the authenticated user on the basis of the stored executability information. The biometric information is information of the user's iris included in an image of the user's eye.

### <Advantageous Effects of Invention>

With the food-processing device according to the present invention, only an authenticated user can execute a process.

With the food-processing device according to the present invention, use of the device by users other than an authenticated user can be suppressed.

With the food-processing device management system and the food-processing device management method according to the present invention, managing information used in the authentication process can be made easier when managing a plurality of food-processing devices having a function of authenticating users.

### BRIEF DESCRIPTION OF THE DRAWINGS

FIG. 1 is a perspective view of an external view of an X-ray inspection device 10 of a first embodiment;
FIG. 2 is a schematic view of an inspection line 100 into which the X-ray inspection device 10 is incorporated;
FIG. 3 is a schematic view of an interior of a shield box 11 of the X-ray inspection device 10;
FIG. 4 is a graph of an example of intensity of penetrative X-rays detected by a line sensor 30;
FIG. 5 is a schematic external view of an iris-imaging device 60;
FIG. 6 is a block diagram of a control device 50;
FIG. 7 is one example of a process information database 71;
FIG. 8 is one example of an iris information database 72;
FIG. 9 is a flowchart representing a procedure of an authentication process of the X-ray inspection device 10;
FIG. 10 is an example of an authentication level database 171 in Modification B;
FIG. 11 is one example of a level-specific process information database 172 in Modification B;
FIG. 12 is a block diagram of a control device 50 in Modification B;
FIG. 13 is one example of a post-authentication process information database 173 in Modification D;
FIG. 14 is a block diagram of the control device 50 in Modification D;
FIG. 15 is a block diagram of the control device 50 in a second embodiment;
FIG. 16 is a drawing for describing an action of a determination part 66 and an authentication effective region R;
FIG. 17 is one example of a beacon terminal information database 73;
FIG. 18 is a flowchart representing a procedure of a user authentication process including an automatic authentication termination process;
FIG. 19 is a diagram of the overall configuration of a food-processing device management system 200 of a third embodiment;
FIG. 20 is a block diagram of the control device 50;
FIG. 21 is a block diagram of a management server 210;
FIG. 22 is a block diagram of the control device 50 in Modification A; and
FIG. 23 is a block diagram of the control device 50 in Modification B.

### DESCRIPTION OF EMBODIMENTS

Embodiments of the present invention are described with reference to the drawings. The embodiment described below is one specific example of the present invention and does not limit the technical range of the present invention.

### <First Embodiment>

### (1) Overall configuration of food-processing device

A food-processing device according to the present invention is a device that performs at least one or more processes on food in a line in which the food is manufactured. Examples of the food-processing device include an X-ray inspection device that detects foreign substances included in the food, a label attachment device that attaches a label to the food, a label inspection device that inspects the label attached to the food, and an allocation device that inspects the food and allocates the food in a plurality of directions in accordance with the inspection results. The food-processing device may optionally be two or more of these devices combined in a coordinated manner. Such a food-processing device is used having been incorporated into, for example, a production line, an inspection line, etc., in a food-manufacturing plant.

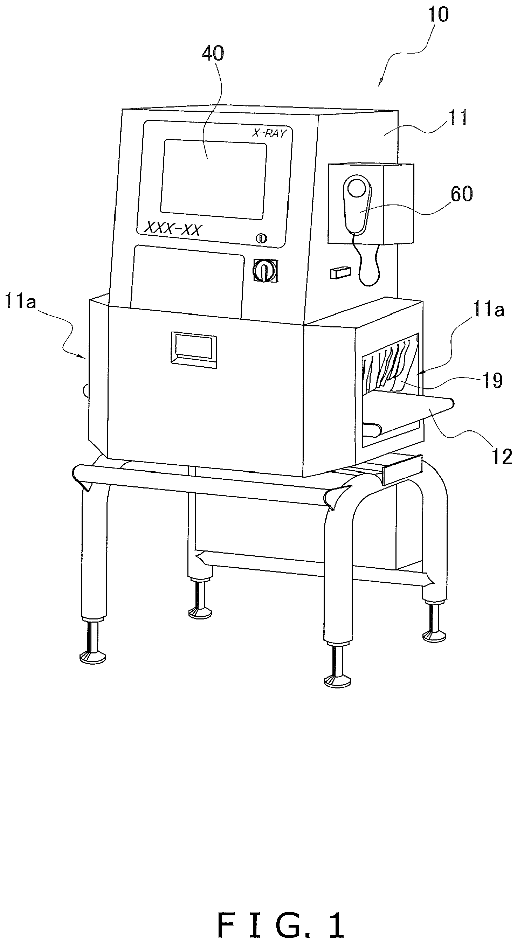

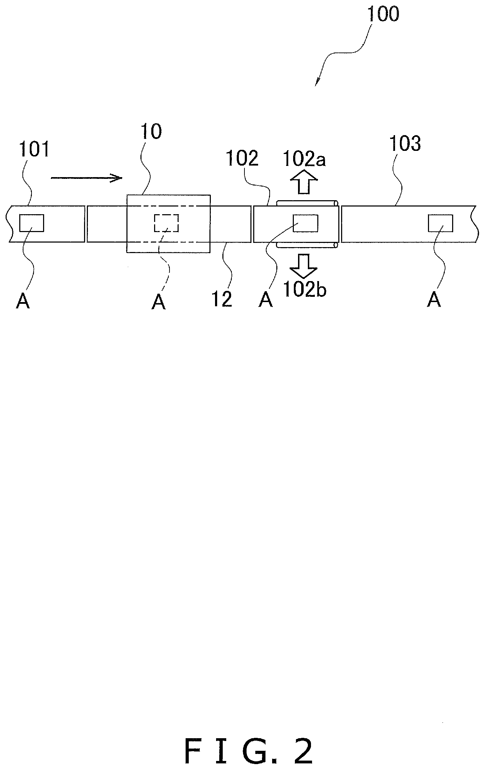

In the present embodiment, the food-processing device is an X-ray inspection device 10. FIG. 1 is a perspective view of an external view of the X-ray inspection device 10. FIG. 2 is a schematic view of an inspection line 100 into which the X-ray inspection device 10 is incorporated. The inspection line 100 performs inspection of a food A, which is an object to be inspected. The food A is, for example, a cooked frozen food packaged in a film. In the inspection line 100, the food A is conveyed by a front conveyor 101 to the X-ray inspection device 10. In FIG. 2, a conveying direction of the food A is shown by an arrow.

The X-ray inspection device 10 performs an acceptability assessment of the food A by using X rays to irradiate the food A being continuously conveyed by the front conveyor 101. Specifically, the X-ray inspection device 10 performs a foreign substance contamination inspection on the food A and classifies the food A as an acceptable item or an unacceptable item on the basis of the inspection result. The inspection result produced by the X-ray inspection device 10 is sent to an allocating mechanism 102 disposed downstream of the X-ray inspection device 10. The allocating mechanism 102 sends food A assessed to be an acceptable item in the X-ray inspection device 10 to a rear conveyor 103 that discharges acceptable items. The allocating mechanism 102 allocates food A assessed to be unacceptable items in the X-ray inspection device 10 to unacceptable item discharge directions 102a, 102b and discharges these foods from the inspection line 100.

### (2) Detailed description of X-ray inspection device

The X-ray inspection device 10 is primarily configured from a shield box 11, a conveying unit 12, an X-ray irradiator 20, a line sensor 30, a monitor 40, an iris-imaging device 60, and a control device 50.

### (2-1) Shield box

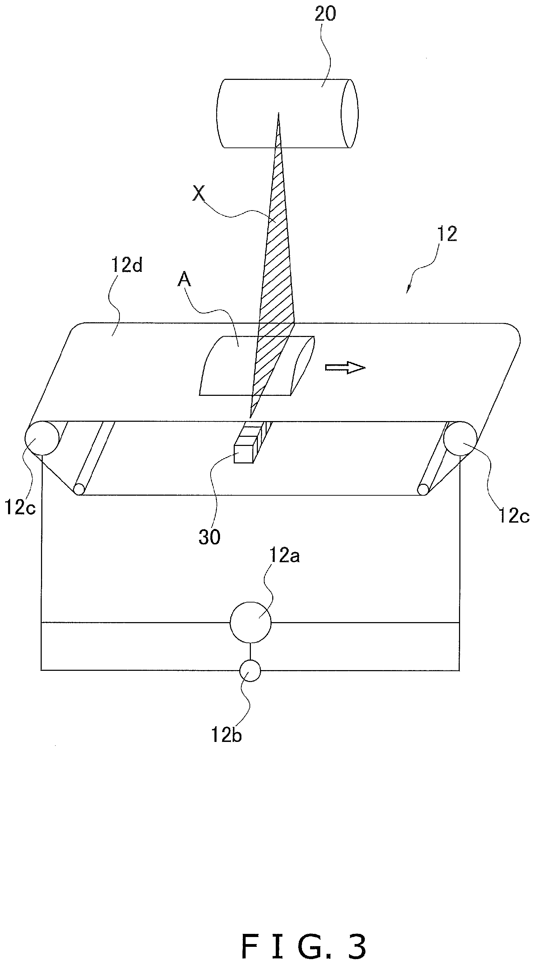

FIG. 3 is a schematic view of an interior of the shield box 11 of the X-ray inspection device 10. The shield box 11 is a casing of the X-ray inspection device 10. Openings 11a for conveying the food A in and out are formed on both side surfaces of the shield box 11, as shown in FIG. 1. The openings 11a are used to convey the food A into the interior from the exterior of the shield box 11, and to convey the food A out to the exterior from the interior of the shield box 11. The openings 11a are covered by shielding curtains 19. The shielding curtains 19 suppress leakage of X-rays from the interior of the shield box 11 to the exterior. The shielding curtains 19 are molded from tungsten sheets. The shielding curtains 19 are pushed apart by the food A when the food A is conveyed in and out.

The conveying unit 12, the X-ray irradiator 20, the line sensor 30, the control device 50, etc., are accommodated inside the shield box 11. The monitor 40, input keys, a power source switch, etc., are disposed in an upper part of a front surface of the shield box 11.

### (2-2) Conveying unit

The conveying unit 12 is a belt conveyor for conveying the food A so as to pass through the interior of the shield box 11. The conveying unit 12 is disposed so as to run through the openings 11a formed on both side surfaces of the shield box 11, as shown in FIG. 1.

The conveying unit 12 is primarily configured from a conveyor motor 12a, an encoder 12b, conveyor rollers 12c, and an endless belt 12d. The conveyor rollers 12c are driven by the conveyor motor 12a. The driving of the conveyor rollers 12c causes the belt 12d to rotate and the food A on the belt 12d to be conveyed. In FIG. 3, a conveying direction of the food A is shown by an arrow.

The speed at which the food A is conveyed by the conveying unit 12 varies depending on a set speed inputted by an operator of the X-ray inspection device 10. The control device 50 performs an inverter control on the conveyor motor 12a on the basis of the set speed, and minutely controls the conveying speed of the food A. The encoder 12b of the conveying unit 12 calculates the conveying speed of the food A by detecting a rotational speed of the conveyor motor 12a, and transmits the conveying speed to the control device 50.

Additionally, the conveying unit 12 uses a belt conveyor as a conveying mechanism, but may optionally use a top chain conveyor, a rotating table, etc., as the conveying mechanism instead of a belt conveyor.

### (2-3) X-ray irradiator

The X-ray irradiator 20 is an X-ray source that uses X-rays to irradiate the food A that has been conveyed by the conveying unit 12 to a prescribed position inside the shield box 11. X-rays radiated from the X-ray irradiator 20 include X-rays of various energies.

The X-ray irradiator 20 is disposed above the conveying unit 12, as shown in FIG. 3. The X-ray irradiator 20 radiates X-rays (radiation light) in a fan shape toward the line sensor 30 disposed below the conveying unit 12. A radiated range X irradiated by the X-rays expands in a direction perpendicular to a conveying surface of the conveying unit 12 and orthogonal to the direction in which the food A is conveyed by the conveying unit 12, as shown in FIG. 3. Namely, the X-rays radiated from the X-ray irradiator 20 expand in a width direction of the belt 12d.

### (2-4) Line sensor

The line sensor 30 is a sensor that detects the X-rays radiated from the X-ray irradiator 20. Specifically, the line sensor 30 detects penetrative X-rays, which are X-rays that penetrate through the food A conveyed by the conveying unit 12.

The line sensor 30 is disposed below the conveying unit 12 as shown in FIG. 3. The line sensor 30 is configured from a plurality of X-ray detection elements. The plurality of X-ray detection elements are disposed horizontally in a straight line along a direction (the width direction of the belt 12d) orthogonal to the direction in which the food A is conveyed by the conveying unit 12.

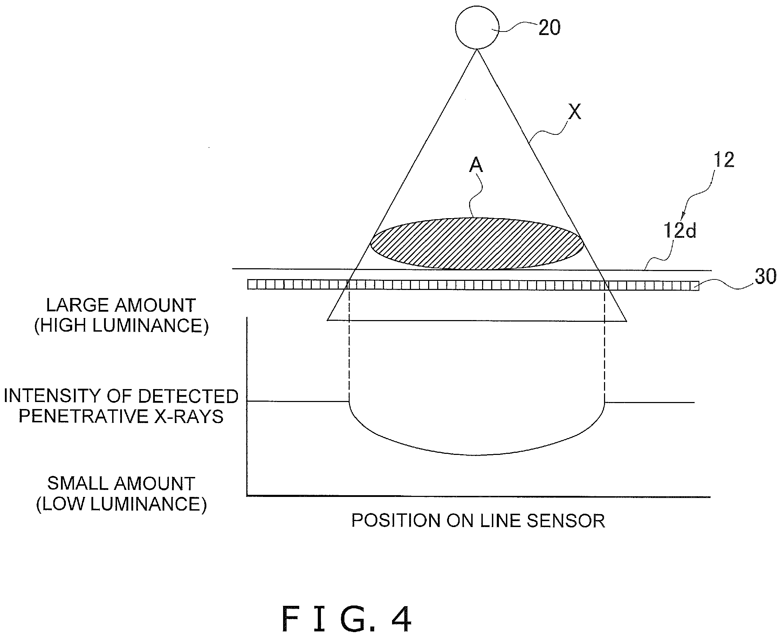

The line sensor 30 detects the penetrative X-rays and outputs an X-ray penetration signal indicating a voltage corresponding to an intensity of the detected penetrative X-rays. The X-ray penetration signal is used to generate a penetration image of the food A, as is described hereinafter. FIG. 4 is a graph of an example of the intensity of the penetrative X-rays detected by the line sensor 30. The horizontal axis of the graph represents positions on the line sensor 30. The vertical axis of the graph represents intensities of the penetrative X-rays detected by the line sensor 30. In the penetration image of the food A, spots where a large amount of penetrative X-rays is detected are displayed brightly (high luminance), and spots where a small amount of penetrative X-rays is detected are displayed dimly (low luminance). Namely, the lightness/dimness (luminance value) of the penetration image of the food A depends on the amount of penetrative X-rays detected. The detected amount of X-rays that have penetrated the food *A* is less than the detected amount of X-rays that have not penetrated the food *A,* as shown in FIG. 4.

The line sensor 30 also functions as a sensor for sensing a timing at which the food *A* passes through the fan-shaped radiated range X (see FIG. 4) of the X-rays. Namely, the line sensor 30 outputs an X-ray penetration signal (first signal) indicating a voltage equal to or less than a prescribed threshold value when the food *A* conveyed by the conveying unit 12 reaches a position above the line sensor 30 (a position overlapping the radiated range X). The line sensor 30 outputs an X-ray penetration signal (second signal) indicating a voltage exceeding the prescribed threshold value when the food *A* passes through the radiated range X. The first signal and the second signal are sent to the control device 50, whereby the presence/absence of the food *A* in the radiated range X is detected.

### (2-5) Monitor

The monitor 40 is a liquid crystal display with a touch panel function. The monitor 40 functions as a display part and an input part of the X-ray inspection device 10. Inspection results, etc., of the food *A* are displayed on the monitor 40. Also displayed on the monitor 40 is a screen image, etc., for inputting initial settings and parameters relating to the acceptability assessment of the food *A.*

The operator of the X-ray inspection device 10 can operate the monitor 40 to input inspection parameters, action setting information, etc. Inspection parameters are parameters required to determine the acceptability of the food *A.* Specifically, inspection parameters are parameters affecting the precision of detecting foreign substances included in the food *A*; e.g., a threshold value, etc., of the intensity of the penetrative X-rays used to determine the presence/absence of foreign substances included in the food *A.* The action setting information is information of an inspection speed of the food *A,* the conveying direction of the conveying unit 12, etc.

The monitor 40 is connected to the control device 50 and sends and receives signals to and from the control device 50. The inspection parameters and the action setting information inputted using the monitor 40 are stored in a storage part 52 of the control device 50.

### (2-6) Iris-imaging device

The iris-imaging device 60 is used to authenticate a user of the X-ray inspection device 10 (hereinafter simply "user"). User authentication is a process that uses the X-ray inspection device 10 to electronically confirm that the user has authority to execute a process related to the manufacture of the food A. The process related to the manufacture of the food *A* is, for example, a process (hereinafter "sensitivity adjustment process") for adjusting sensitivity for determining the acceptability of the food *A*, using the X-ray inspection device 10. In this case, the user must be authenticated using the iris-imaging device 60 in order to execute the sensitivity adjustment process of the X-ray inspection device 10.

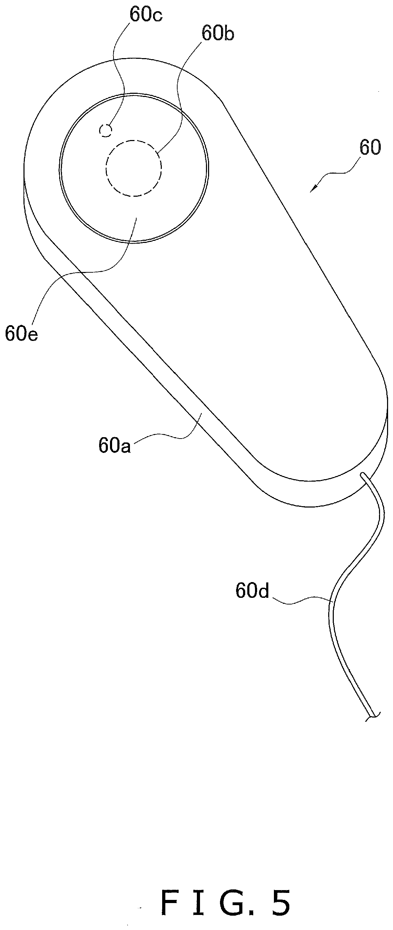

The iris-imaging device 60 is a non-contact kind of biometric authentication device, which utilizes an image of an iris of an eye of the user. FIG. 5 is a schematic external view of the iris-imaging device 60. The iris-imaging device 60 primarily has a portable casing 60a, an infrared camera 60b, an infrared illumination 60c, and a connecting cable 60d. The iris-imaging device 60 is installed in a position the user can easily access when operating the X-ray inspection device 10, such as near the monitor 40, as shown in FIG. 1.

The portable casing 60a houses the infrared camera 60b and the infrared illumination 60c, and is connected to a body of the X-ray inspection device 10 via the connecting cable 60d. The portable casing 60a can be attached to and detached from a bracket (not shown) attached to the shield box 11. When using the iris-imaging device 60, the user can remove the portable casing 60a from the shield box 11 as necessary and hold the portable casing 60a.

The infrared camera 60b is a camera capable of sensing infrared rays. The infrared illumination 60c is an LED that radiates low-intensity infrared rays. The infrared camera 60b and the infrared illumination 60c are protected by a protective cover 60e. The protective cover 60e is a transparent plastic plate, glass plate, etc.

The connecting cable 60d is a USB cable, etc. The connecting cable 60d is a connecting cable for connecting the infrared camera 60b and the infrared illumination 60c with the control device 50 so as to enable mutual communication. The infrared camera 60b and the infrared illumination 60c may also optionally be connected wirelessly with the control device 50. In this case, the iris-imaging device 60 does not need to have the connecting cable 60d.

Before authenticating themselves using the iris-imaging device 60, the user must pre-register biometric information relating to their iris. The registration process is described hereinafter. When using the iris-imaging device 60 to authenticate themselves, the user removes the portable casing 60a from the bracket of the shield box 11 to hold the portable casing 60a, and brings their eye and the protective cover 60e close together. At this time, the infrared illumination 60c radiates infrared rays. The infrared rays radiated from the infrared illumination 60c pass through the protective cover 60e and reflect off the user's eye. The infrared camera 60b captures an image of the user's eye by sensing the infrared rays that have reflected off the user's eye and passed back through the protective cover 60e. The image of the user's eye acquired by the infrared camera 60b is sent to the control device 50 via the connecting cable 60d and used in the user authentication process.

### (2-7) Control device

The control device 50 is primarily configured from a CPU, a ROM, a RAM, a hard disk drive (HDD), etc. A solid state drive (SSD) may optionally be used instead of an HDD. The control device 50 is also provided with a display control circuit, an input circuit, a communication port (none of which are shown), etc. The display control circuit is a circuit that controls a display on the monitor 40. The input circuit is a circuit that takes in input data inputted by the operator via the touch panel and input keys of the monitor 40. The communication port is a port that enables connection with a printer or other external machinery and a LAN or other network.

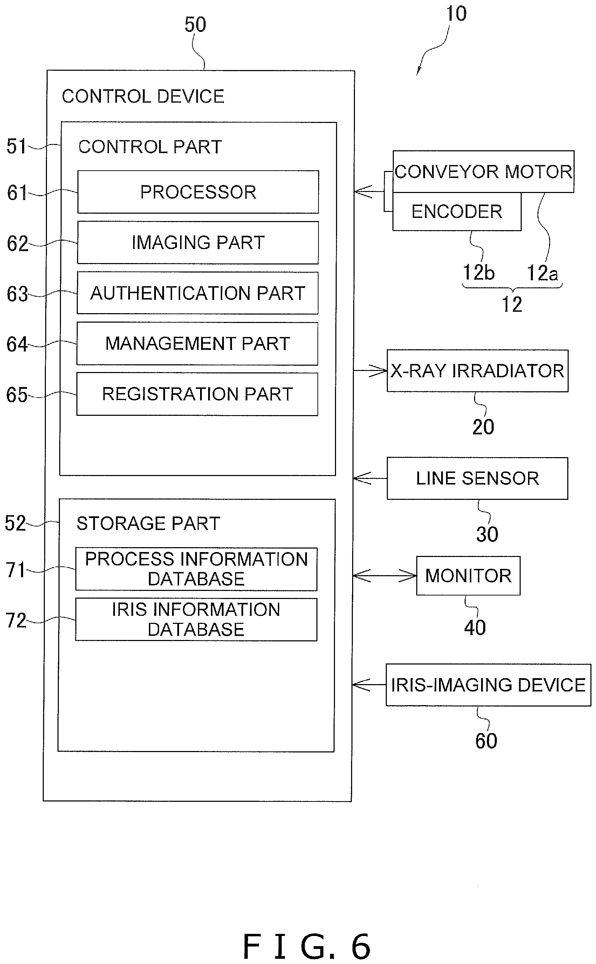

FIG. 6 is a block diagram of the control device 50. The control device 50 primarily has a control part 51 and the storage part 52. The control device 50 is electrically connected to the conveyor motor 12a, the encoder 12b, the X-ray irradiator 20, the line sensor 30, the monitor 40, the iris-imaging device 60, etc.

### (2-7-1) Control part

The control part 51 primarily has a processor 61, an imaging part 62, an authentication part 63, a management part 64, and a registration part 65. These are each a function realized by executing a program stored in the storage part 52.

### (2-7-1-1) Processor

The processor 61 controls the conveying unit 12, the X-ray irradiator 20, and the line sensor 30, determines the presence/absence of foreign substances included in the food *A,* and determines the acceptability of the food *A.* Specifically, the processor 61 firstly acquires data pertaining to the rotational speed of the conveyor motor 12a from the encoder 12b, and calculates a movement distance of the food *A* on the basis of this data. Next, the processor 61 receives an X-ray penetration signal outputted from the line sensor 30 and detects a timing at which the food *A* on the belt 12d of the conveying unit 12 has arrived in the radiated range X of the X-rays. Next, the processor 61 determines the presence/absence of foreign substances included in the food *A* on the basis of the intensity of the penetrative X-rays and determines the acceptability of the food *A.* The processor 61 also displays the determination result of the acceptability of the food A on the monitor 40.

The processor 61 executes the sensitivity adjustment process of the X-ray inspection device 10 as performed by the authenticated user. The sensitivity adjustment process shall be described here. In order for foreign substances included in the food *A* to be detected with high precision using the X-ray inspection device 10, a sensitivity adjustment process for optimizing a foreign substance detection precision must be performed by means of a pre-adjustment or a periodic check. For that reason, in order to guarantee safety and quality of the food *A,* the X-ray inspection device 10 preferably has a configuration such that when the sensitivity adjustment process is not performed in advance by a user having special authority, the foreign substance contamination inspection of the food *A* cannot be started by a user not having special authority. However, in the case of such a configuration, when authentication for allowing the sensitivity adjustment process to be executed is performed on the basis of a password, an IC card, etc., a situation could arise in which a user not having special authority is authenticated. In this case, there is a possibility of a problem arising in which a user not having special authority performs the sensitivity adjustment process in order to enable starting the foreign substance contamination inspection. In view of this, in the X-ray inspection device 10, the user is authenticated on the basis of biometric information intrinsic to each user; namely, an image of an eye captured using the iris-imaging device 60, as is described hereinafter. The X-ray inspection device 10 can thereby reliably authenticate the user, and sensitivity adjustment operations and other critical operations can be executed only when the user has been authenticated. As a result, there could not be a situation in which a user not having special authority is authenticated; therefore, occurrences of problems such as reinspection or recovery of food that might not have been appropriately inspected, resulting from the malfunction of foreign substance detection errors (erroneous detection, detection leakage, etc.) when the sensitivity adjustment process has not been appropriately performed, are suppressed.

### (2-7-1-2) Imaging part

The imaging part 62 controls the iris-imaging device 60 to capture an image of the user's eye. Specifically, the imaging part 62 first senses that the user authentication process performed by the iris-imaging device 60 has been started. The start of the authentication process is sensed by, for example, the imaging part 62 detecting that the iris-imaging device 60 has been removed from the bracket of the shield box 11, or by the user inputting the starting of the authentication process via the monitor 40, etc. Next, upon sensing the start of the authentication process, the imaging part 62 radiates infrared rays from the infrared illumination 60c. Next, when the user brings their eye and the protective cover 60e of the iris-imaging device 60 close together, the imaging part 62 senses the infrared rays reflected off the user's eye using the infrared camera 60b, and acquires an image of the user's eye. Next, the imaging part 62 sends digital data of the acquired image of the user's eye to the control device 50 from the infrared camera 60b.

### (2-7-1-3) Authentication part

The authentication part 63 performs the user authentication process on the basis of the data of the image of the user's eye acquired and transmitted by the imaging part 62. The user authentication process is performed before the start of the use of the X-ray inspection device 10. The user is able to execute the sensitivity adjustment process of the X-ray inspection device 10 only when authenticated by the authentication part 63. Specifically, the authentication part 63 first extracts a section corresponding to the user's iris from the image of the user's eye. Next, the authentication part 63 converts an image of only the section of the user's iris to iris information of the user, using a dedicated algorithm. The iris information is a bit string of a prescribed length which can be utilized as biometric authentication information.

Next, the authentication part 63 determines whether or not the iris information (during-authentication iris information) converted from the image of the user's eye before the start of the use of the X-ray inspection device 10 is included in iris information (registered iris information, described hereinafter) that has been registered in advance in the registration part 65. If the during-authentication iris information is included in the registered iris information, the authentication part 63 determines that the user has the authority to execute the sensitivity adjustment process of the X-ray inspection device 10. If the during-authentication iris information is not included in the registered iris information, the authentication part 63 determines that the user does not have the authority to execute the sensitivity adjustment process of the X-ray inspection device 10. An authenticated user is a user determined by the authentication part 63 to have the authority to execute the sensitivity adjustment process of the X-ray inspection device 10.

### (2-7-1-4) Management part

The management part 64 makes a process performed by the processor 61 executable for the user authenticated by the authentication part 63. Specifically, when the authentication part 63 authenticates the user, the management part 64 controls the X-ray inspection device 10 so that the authenticated user is able to start the sensitivity adjustment process performed by the processor 61. For example, the management part 64 displays on the monitor 40 a screen image for executing the sensitivity adjustment process when the authentication part 63 has authenticated a user.

### (2-7-1-5) Registration part

The registration part 65 registers data pertaining to the image of the user's eye. Specifically, before the start of the use of the X-ray inspection device 10, the registration part 65 registers iris information converted from the image of each user's eye for all users that could possibly use the X-ray inspection device 10. The registration part 65 registers user identification information and the registered iris information in correlation in an iris information database 72 (described hereinafter). The user identification information is an ID number for uniquely identifying a user. This ID number is, for example, a character string configured from a plurality of alphanumeric characters. The registered iris information is a bit string that is converted from the image of the user's eye captured in advance by the imaging part 62, and that can be utilized as biometric authentication information.

### (2-7-2) Storage part

The storage part 52 primarily has a process information database 71 and the iris information database 72. These databases are stored in an HDD or an SSD.

### (2-7-2-1) Process information database

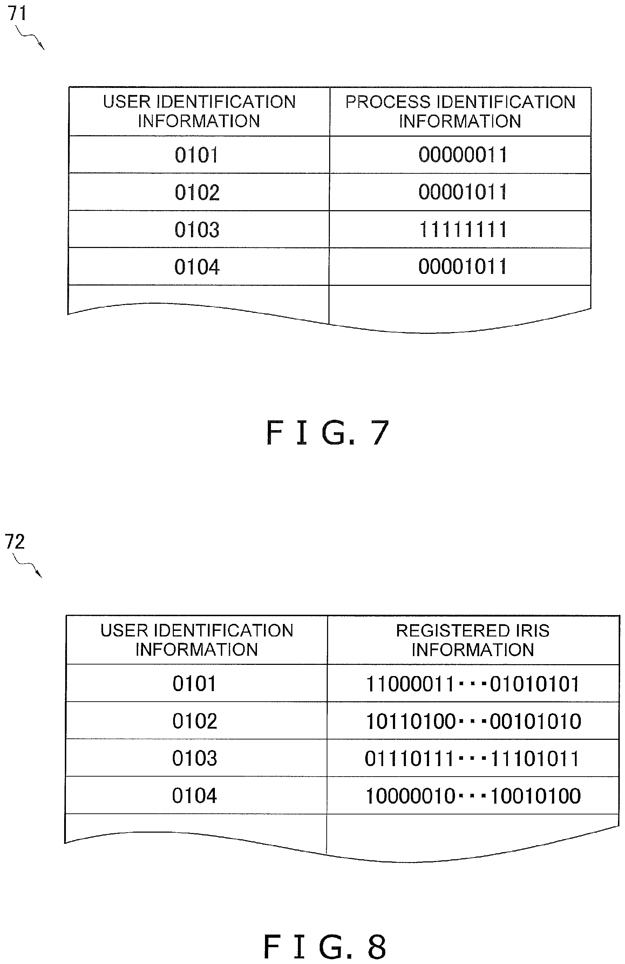

FIG. 7 is one example of the process information database 71. The process information database 71 correlates and stores users and processes made executable for the users by the management part 64. Specifically, the process information database 71 is a database in which the user identification information and process identification information are correlated, and the user identification information is set as a main key. The user identification information is an ID number for uniquely identifying the user. The process identification information is a bit string representing processes executable on the X-ray inspection device 10 by the user when the user has been authenticated by the authentication part 63. Each bit of the process identification information represents a corresponding "executable process."

The process information database 71 is, for example, created in advance before the start of the handling of the X-ray inspection device 10 by a manager, etc., of the X-ray inspection device 10 or the inspection line 100, and is revised after the start of the handling of the X-ray inspection device 10.

### (2-7-2-2) Iris information database

FIG. 8 is one example of the iris information database 72. The iris information database 72 correlates and stores users and iris information (registered iris information) of the users registered in advance using the registration part 65. Specifically, the iris information database 72 is a database in which the user identification information and the registered iris information are correlated, and the user identification information is set as a main key. The user identification information is an ID number shared with the user identification information used by the process information database 71. The registered iris information is a bit string that is converted from the image of the user's eye and that can be utilized as biometric authentication information.

The iris information database 72 is, for example, created in advance before the start of the handling of the X-ray inspection device 10 by a manager, etc., of the X-ray inspection device 10 or the inspection line 100, and is revised after the start of the handling of the X-ray inspection device 10.

### (3) Authentication process of X-ray inspection device

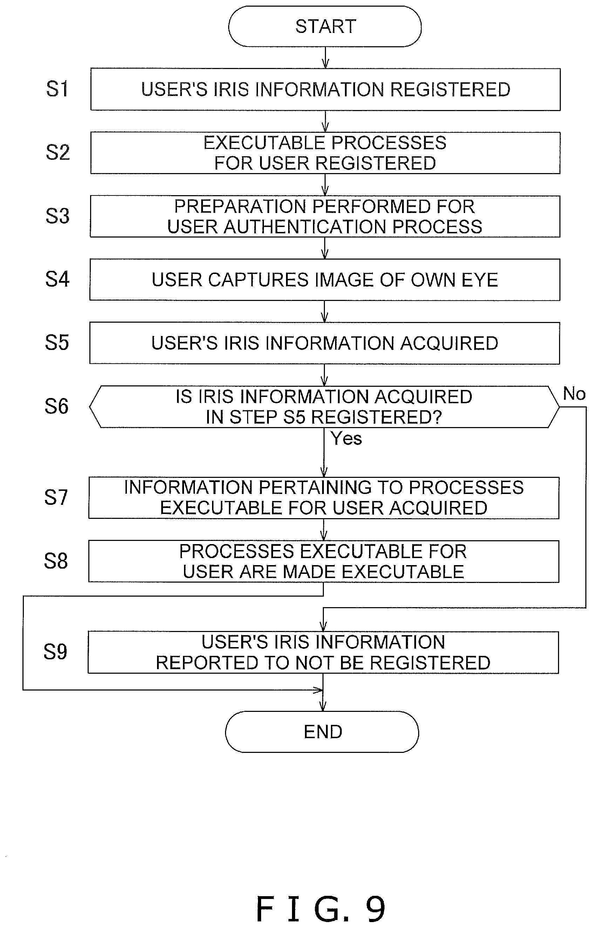

FIG. 9 is a flowchart representing a procedure of the authentication process of the X-ray inspection device 10. Steps S1 and S2 pertain to the iris information registration process, which is performed in advance before the start of the authentication process of the X-ray inspection device 10. Steps S1 and S2 pertain to advance preparations and can be executed in any order. Steps S3 to S9 pertain to the user authentication process, which is performed before the start of the use of the X-ray inspection device 10.

In step S1, the user's iris information is registered. In step S1, each user of the X-ray inspection device 10 captures images of their eye using the iris-imaging device 60, and registers their iris information in the iris information database 72 in advance. This operation is performed by all users who could possibly use the X-ray inspection device 10, before the sensitivity adjustment process of the X-ray inspection device 10 is started.

In step S2, information pertaining to processes executable on the X-ray inspection device 10 by users is registered in the process information database 71 for the users registered in the iris information database 72 in step S1. This operation is normally performed by the manager of the X-ray inspection device 10 or the inspection line 100. The manager is able to operate the monitor 40 of the X-ray inspection device 10 or a server, etc., connected to the X-ray inspection device 10, input the information pertaining to the processes executable by each user, and collectively register this information in the process information database 71.

In step S3, preparation is performed for the user authentication process performed by the X-ray inspection device 10. This is performed immediately before a user starts to use the X-ray inspection device 10. The user is thereby able to execute the authentication process of the X-ray inspection device 10.

In step S4, the user captures an image of their eye using the iris-imaging device 60. Specifically, the user removes the iris-imaging device 60 from the shield box 11 of the X-ray inspection device 10 and brings the protective cover 60e of the iris-imaging device 60 close to their eye. The iris-imaging device 60 thereby automatically captures an image of the user's eye.

In step S5, the user's iris information is acquired on the basis of the image of the user's eye captured in step S4. The processes from step S5 onward are performed automatically.

In step S6, a determination is made as to whether or not the user's iris information acquired in step S5 is included in the iris information database 72 registered in step S1. If the determination result is `YES', the user is authenticated and a transition is made to step S7. If the determination result is 'NO', the user is not authenticated and a transition is made to step S8.

In step S7, information pertaining to processes executable by the user authenticated in step S6 is acquired from the process information database 71 registered in step S2.

In step S8, the processes executable by the user, which were acquired in step S7, are put into a state of actually being executable on the X-ray inspection device 10 by the user. At this time, the user authenticated in step S6 and the processes acquired in step S7 and executable by the user may optionally be displayed on the monitor 40. The user is thereby able to refer to the monitor 40 to confirm the information pertaining to their authenticated self (name, department, etc.) and the processes executable by the user. From this point onward, the authenticated user is able to execute the sensitivity adjustment process of the X-ray inspection device 10.

In step S9, the user is given a report that, *inter alia,* the iris information of the user not authenticated in step S6 has not been registered in the iris information database 72. For example, the monitor 40 displays a warning message stating that, *inter alia,* "The device cannot be operated because the iris information has not been registered."

### (4) Characteristics

(4-1)
   The X-ray inspection device 10, which is a food-processing device according to the present embodiment, utilizes an image of a user's eye as biometric information for the user authentication process. Situations in which a user is authenticated who should not be, and situations in which the authenticated user is not the same person as the actual user, are thereby avoided. Consequently, only an authenticated user is able to execute a process with the X-ray inspection device 10.
(4-2)
   The X-ray inspection device 10 utilizes, as biometric information for the user authentication process, iris information obtained from the iris image data included in the image of the user's eye. Biometric authentication using the user's iris has a high identification capability similar to biometric authentication using the user's fingerprint, but is particularly suitable for the authentication process because of advantages such as the following. Firstly, the pattern of a human iris is complicated and is therefore suitable as information for specifying a user. Secondly, the pattern of a human iris mostly does not change even after a long period of time and is substantially impossible to falsify; therefore, iris information once registered can be utilized semi-permanently as highly reliable biometric information. Thirdly, non-contact authentication processes can be performed merely by capturing an image of the user's eye using a comparatively small device such as the iris-imaging device 60; therefore, there is no need to ensure a large space to install special instruments for the authentication process. Therefore, the X-ray inspection device 10 is capable of executing a highly precise and highly reliable authentication process.
(4-3)
   In a food-manufacturing plant, etc., in which the X-ray inspection device 10 is installed, users wear operational clothing that covers parts other than the eyes in order to prevent hair from becoming mixed into the food *A* and skin bacteria from adhering to the food *A.* In this case, of the parts of the user's body, only the user's eyes are exposed to the exterior. For that reason, information obtained from the image of the user's eye is used as biometric information capable of specifying the user, whereby the user is able to perform the authentication process and start using the X-ray inspection device 10 while wearing the operational clothing. Namely, the user need not remove the operational clothing to expose a part of the body in order to perform the authentication process; therefore, when the authentication process is performed, occurrences of problems in which the user's hair becomes mixed into the food *A* or bacteria of the user's skin adheres to the food *A* are suppressed. Therefore, the X-ray inspection device 10 is particularly useful in a food-manufacturing plant, etc., in which foreign substances must be prevented as much as possible from becoming mixed into and adhering to the food in order to guarantee product safety and quality.
(4-4)
   The storage part 52 of the X-ray inspection device 10 has the process information database 71, which correlates and stores users and processes that become executable for the users after authentication. For that reason, the manager (user having special authority) of the X-ray inspection device 10 is able to operate the process information database 71 to collectively set and manage the processes executable by each user in advance. Situations in which, for example, a user executes critical operations that only a manager should execute are thereby avoided. Such critical operations are, for example, an operation adjusting the detection precision for foreign substances included in the food *A,* and such operations must be performed by a manager having the required skill and qualifications in order to guarantee safety and quality of the food *A.*
(4-5)
   The storage part 52 of the X-ray inspection device 10 has the iris information database 72, which correlates and stores users and iris information obtained from images of the users' eyes. The manager of the X-ray inspection device 10 can thereby operate the iris information database 72 to easily perform the processes of registering and referencing the iris information for each user.

### (5) Modifications

Although one embodiment of the present invention is described above, this embodiment is not provided by way of limitation on the present invention, and various changes can be made within a range that does not deviate from the scope of the invention.

### (5-1) Modification A

In the embodiment, the storage part 52 of the X-ray inspection device 10 has the iris information database 72, which correlates and stores users and the iris information acquired from images of the users' eyes. However, the storage part 52 of the X-ray inspection device 10 may optionally have, instead of the iris information database 72, a database that correlates and stores users and image data of the users' eyes. In this database, for example, user identification information serving as a main key and eye image data or an address (save location) of the image data are correlated for each user.

In the embodiment, the iris-imaging device 60 has a function for capturing an image of the user's eye, but may also optionally have a function for capturing a moving image of the user's eye during imaging. In this case, the X-ray inspection device 10 may optionally have, instead of the iris information database 72, a database that correlates and stores users and moving image data of the users' eyes. In this database, for example, user identification information serving as a main key and eye moving image data or an address of the moving image data are correlated for each user.

In the present modification, instead of the user's iris information being registered in step S1 in FIG. 9 of the embodiment, image data or moving image data (or an address thereof) of the user's eye is registered. In step S6 in FIG. 9, a determination is made as to whether or not the user's iris information acquired in step S5 is included in iris information acquired from the image data or moving image data of the eye registered in step S1.

### (5-2) Modification B

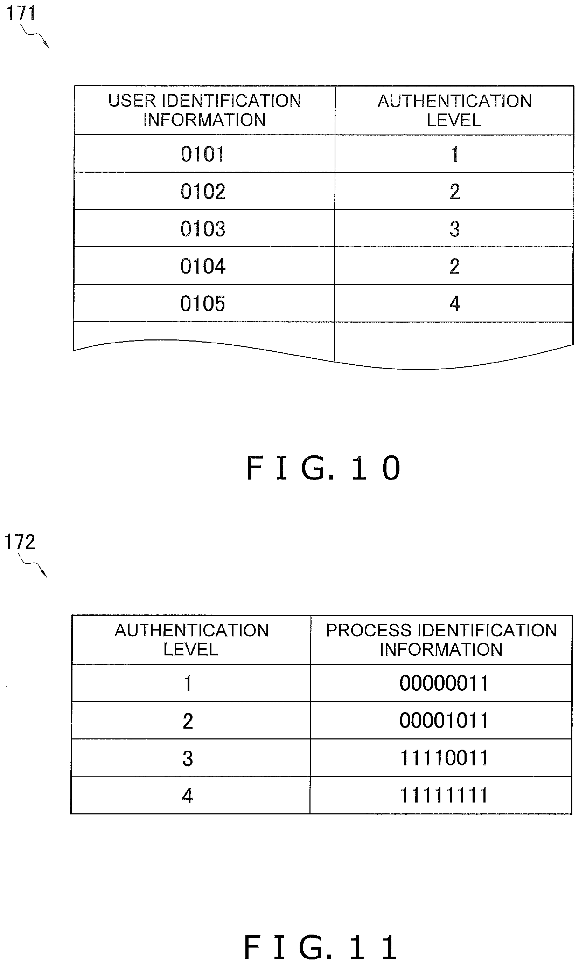

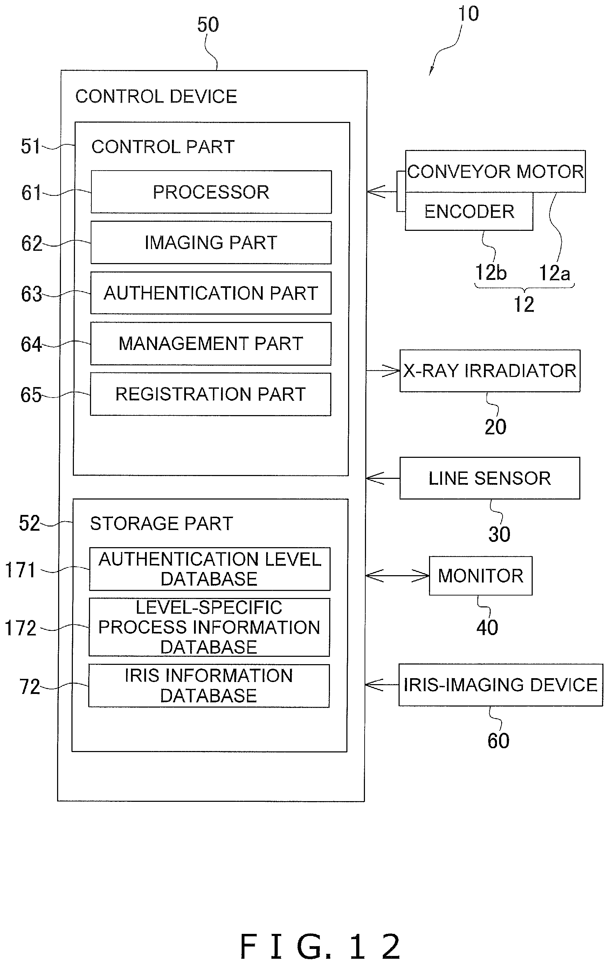

In the embodiment, the storage part 52 of the X-ray inspection device 10 has the process information database 71, which correlates and stores users and processes that become executable for the users after authentication. However, the storage part 52 of the X-ray inspection device 10 may optionally have, instead of the process information database 71, an authentication level database 171 and a level-specific process information database 172. FIG. 10 is one example of the authentication level database 171. FIG. 11 is one example of the level-specific process information database 172. FIG. 12 is a block diagram of the control device 50 in the present modification.

The authentication level database 171 correlates and stores users and authentication levels. In FIG. 10, user identification information serving as a main key and an authentication level are correlated for each user. The authentication level correlated with each user is only one of a plurality of authentication levels. In this modification, the lowest authentication level is 1 and the highest authentication level is 4. The authentication level represents the level of authority granted to the user.

For example, a user whose authentication level is 1 can execute only a process of the lowest limit required to determine the acceptability of the food *A* using the X-ray inspection device 10. A user whose authentication level is 2 is able to further execute a process controlling the action setting information such as the inspection speed of the food *A,* and the conveying direction of the conveying unit 12. A user whose authentication level is 3 is able to further execute a process in a critical control point (CCP) of a food-manufacturing step. In this modification, the process in the critical control point (CCP) of the food-manufacturing step is, for example, the sensitivity adjustment process of the embodiment or another process controlling inspection parameters affecting the foreign substance detection precision; an operation recording (periodic) measurement results of a temperature of the food *A* and an operation adjusting a measurement precision thereof; and an operation recording (periodic) measurement results of a concentration of a harmful chemical substance adhering to the food *A* and an operation adjusting a measurement precision thereof, etc. A user whose authentication level is 4 is able to further start up a special action mode of the X-ray inspection device 10. The special action mode is, for example, a mode for performing countermeasure and maintenance checkup operations during an emergency of the X-ray inspection device 10, and a mode for performing an operation registering a user having special authority when delivering the X-ray inspection device 10. In the present modification, a user granted any of the authentication levels 1 to 4 must perform an authentication process using iris information included in an image of their eye in order to perform processes pertaining to the manufacture of the food *A* using the X-ray inspection device 10.

The level-specific process information database 172 correlates and stores authentication levels and processes that become executable upon authentication. In FIG. 11, an authentication level serving as a main key and process identification information are correlated for each authentication level. The process identification information, as in the embodiment, is a bit string representing processes that become executable upon authentication.

In the present modification, the authentication part 63 acquires information pertaining to a process executable by the user authenticated in step S6, this information being acquired in step S7 in FIG. 9 of the embodiment on the basis of the authentication level database 171 and the level-specific process information database 172. Specifically, the authentication part 63 first acquires the authentication level of the authenticated user on the basis of the authentication level database 171. Next, the authentication part 63 acquires, on the basis of the level-specific process information database 172, information corresponding to the authentication level of the authenticated user and pertaining to the process executable by the user.

In the present modification, the manager of the X-ray inspection device 10 is able to sort users into a plurality of groups by authentication level and set processes executable by the users for each group. Namely, the manager of the X-ray inspection device 10 need not set processes executable by the user for each user. For that reason, with the X-ray inspection device 10 of the present modification, processes executable by the users can be easily managed by setting the executable processes in advance for each authentication level and setting an authentication level for each user.

### (5-3) Modification C

In Modification B, the storage part 52 of the X-ray inspection device 10 has the authentication level database 171 and the level-specific process information database 172. Information pertaining to the process executable by the authenticated user is acquired in the authentication part 63 on the basis of the authentication level database 171 and the level-specific process information database 172.

However, in Modification B, the authentication part 63 may optionally use information other than the image of the user's eye as the basis to perform the authentication process for a user for whom the highest of the plurality of authentication levels has been set. In the authentication level database 171 shown in FIG. 11, the highest authentication level is 4. A user whose authentication level is 4 is able to execute processes that only a person having special skill and qualification has the authority to execute.

In the present modification, a user for whom the highest authentication level is set (a user having special authority) is able to perform not only an authentication process by using iris information included in an image of their eye, but also an authentication process by using a method involving information other than iris information. For example, a user having an authentication level of 4 is able to perform an authentication process by using the monitor 40 to input a dedicated password and is able to activate the X-ray inspection device 10 in a dedicated mode to perform countermeasure and maintenance checkup operations during an emergency. For that reason, with the X-ray inspection device 10 of the present modification, even during an emergency such as, for example, when a malfunction has occurred in the iris-imaging device 60, etc., a user for whom the highest authentication level has been set is able to execute the sensitivity adjustment process as one example of a countermeasure during an emergency, and is also able to execute maintenance checkup operations, etc., without performing the authentication process using iris information. In this case, information pertaining to the user who performed the authentication process with a method other than the authentication process using iris information, information pertaining to the process executed by that user, etc., may optionally be automatically stored in the storage part 52.

### (5-4) Modification D

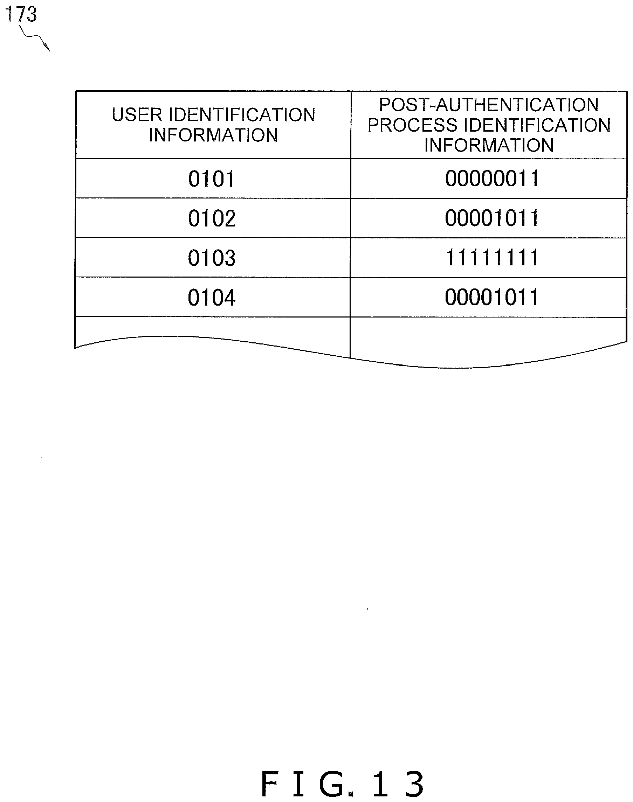

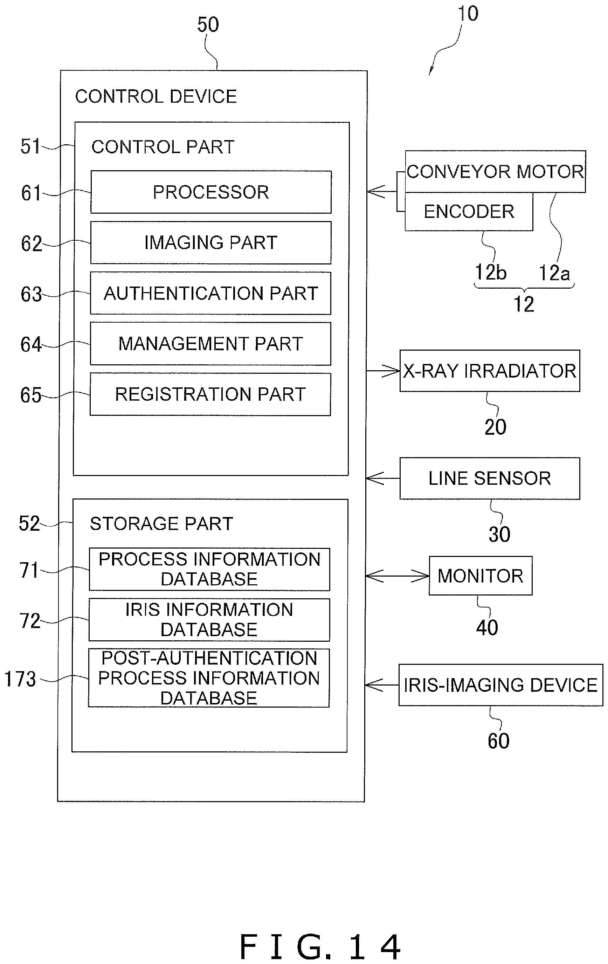

The storage part 52 of the X-ray inspection device 10 may also optionally be provided with a post-authentication process information database 173. FIG. 13 is one example of the post-authentication process information database 173. FIG. 14 is a block diagram of the control device 50 in the present modification. The post-authentication process information database 173 correlates and stores users authenticated by the authentication part 63 and processes made executable for the users by the management part 64. Specifically, the post-authentication process information database 173 correlates user identification information serving as a main key and post-authentication process identification information for each user. The post-authentication process identification information represents processes that become executable for the user after the user has been authenticated. The post-authentication process identification information, as with the process identification information of the embodiment, is a bit string representing processes performed by the processor 61.

In the present modification, the X-ray inspection device 10 stores authenticated users and post-authentication operation specifics of the users in correlation in the post-authentication process information database 173 in advance, whereby the traceability of the food A can be improved. The operation specifics include not only information representing executed processes (process IDs, etc.), but also data (values) acquired during the processes, the results of the processes, etc.

In the present modification, the control device 50 preferably prohibits changes and deletions of the record included in the post-authentication process information database 173. Namely, the post-authentication process information database 173 is preferably configured so that additions to the record are allowed but changes and deletions of the record are prohibited. In this case, falsification of the post-authentication process information database 173 is prevented and the reliability of the information stored in the post-authentication process information database 173 is therefore improved. The control device 50 may optionally be configured so that the record can be deleted upon fulfilment of conditions by which record preservation is guaranteed, in cases such as when the transfer of the record to another recording medium is complete.

### (5-5) Modification E

In step S6 in FIG. 9 of the embodiment, a determination is made as to whether or not the user's iris information acquired in step S5 is included in the iris information database 72 registered in step S1. However, in step S6 in FIG. 9, the rate of concordance between the user's iris information (during-authentication iris information) acquired in step S5 and the iris information (registered iris information) registered in step S1 and included in the iris information database 72 may optionally be used as a basis to determine whether or not the during-authentication iris information is included in the registered iris information. Specifically, the authentication part 63 may optionally compare a bit string of the during-authentication iris information and a bit string of the registered iris information, and determine that the during-authentication iris information is included in the registered iris information and authenticate the user when the rate of concordance of the bits is equal to or greater than a prescribed value.

### (5-6) Modification F

In the embodiment, the user can detach the iris-imaging device 60 from the bracket of the shield box 11. For that reason, the user can bring the iris-imaging device 60 removed from the bracket close to their eye to capture an image of their eye. However, the X-ray inspection device 10 may optionally have a mechanism capable of adjusting at least one of a position and an orientation of the iris-imaging device 60. In this case, the user can capture an eye image without removing the iris-imaging device 60 from the bracket, by adjusting the position and the orientation of the iris-imaging device 60 in accordance with a position of their eye. Therefore, in the present modification, the X-ray inspection device 10 can easily acquire an image of the user's eye in accordance with the user's height.

### (5-7) Modification G

In the embodiment, the management part 64 of the X-ray inspection device 10 makes a process performed by the processor 61 executable for a user authenticated by the authentication part 63. However, when a prescribed time has elapsed after the user was authenticated by the authentication part 63, the management part 64 may optionally render the process performed by the processor 61 unexecutable until the user has again been authenticated by the authentication part 63. The prescribed time may optionally be set to be a fixed length of time, the time required for the process performed by the processor 61, or a time such as the time for which the X-ray inspection device 10 remained unoperated. In this case, even when the authentication process is performed and the foreign substance contamination inspection becomes executable, if the prescribed time has elapsed since the authentication, the user cannot proceed with the foreign substance contamination inspection without performing the authentication process. Due to this configuration, occurrences of the malfunction of another user being able to execute the foreign substance contamination inspection without performing the authentication process based on an image of their eye while the user authenticated by the authentication process is temporarily away from the X-ray inspection device 10 are suppressed. Namely, in the present modification, the X-ray inspection device 10 is capable of suppressing instances in which an actual user continues to be able to execute the process when the authenticated user is a different person from the actual user.

Additionally, when, for example, the user of the X-ray inspection device 10 performs an operation adjusting the foreign substance detection precision after being authenticated and another adjustment operation is performed after the first adjustment operation has been completed, it is sometimes unclear whether or not the second and subsequent instances of the adjustment operation were performed by the authenticated user. For that reason, in the present modification, when a user authenticated by the authentication part 63 has performed a specific process (e.g., an operation adjusting the foreign substance detection precision), the management part 64 may optionally render this specific process performed by the processor 61 unexecutable until this user is again authenticated by the authentication part 63. Due to this configuration, in the example described above, the user of the X-ray inspection device 10 must perform the authentication process every time the user performs the operation adjusting the foreign substance detection precision, and instances in which the adjustment operation is performed by a user not having special authority are therefore avoided.

### (5-8) Modification H

In the embodiment, the X-ray inspection device 10 is a food-processing device that performs a foreign substance contamination inspection for a food *A*, and is a device that performs one process (foreign substance contamination inspection) on the food A, which is an object to be inspected. However, the food-processing device may optionally be a device that performs a plurality of processes continuously or in parallel. For example, the food-processing device may be configured from the X-ray inspection device 10 and a label attachment device positioned downstream of the X-ray inspection device 10. The X-ray inspection device 10 and the label attachment device are positioned near each other on the production line of the food A, and can therefore be used simultaneously by the same user. For that reason, the food-processing device has a configuration in which a user performs the authentication process of one device, either the X-ray inspection device 10 or the label attachment device, and the authentication process of the other device is thereby performed. Similarly, even in cases in which the food-processing device is configured from three or more processing devices, the food-processing devices may have a configuration in which a user performs the authentication process of any one processing device and the authentication processes of all the other processing devices are thereby performed.

### (5-9) Modification I

In the embodiment, the food-processing device according to the present invention is the X-ray inspection device 10, which uses X-rays to perform the foreign substance contamination inspection of the food *A.* However, the food-processing device according to the present invention is not limited to the X-ray inspection device 10. For example, the food-processing device may optionally be a foreign substance inspection device that uses light (infrared rays, ultraviolet rays, visible light, etc.) and that is used in the food-manufacturing step, a device for managing the temperature of the food *A,* a device for managing a supply of medicine (such as aqueous hypochlorous acid) used in the food *A,* and a device for printing labels to be attached to the food *A.*

When the food-processing device is a label-printing device, a process requiring user authentication is, for example, the process of changing and deciding the information to be printed on a label (information pertaining to the food, including at least one of the following: product name, contents, amount contained, expiration date, price, allergy indications, etc.). Since the food *A* must be recalled when the information printed on the label is not appropriate, this process must be performed by a user having the prescribed authority or a user having the prescribed authentication level in Modification B.

### <Second Embodiment>

A food-processing device of the present embodiment resembles the food-processing device of the first embodiment in terms of basic configuration and actions. The food-processing device is an X-ray inspection device 10 that uses X-rays to perform a foreign substance contamination inspection on a food *A.* Differences between the X-ray inspection device 10 of the present embodiment and the X-ray inspection device 10 of the first embodiment are described below.

### (1) Configuration

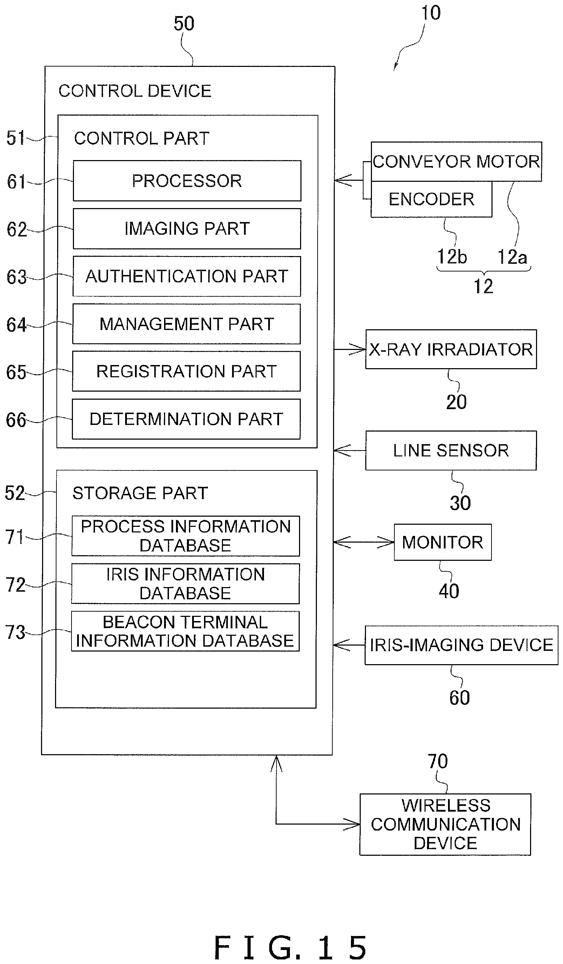

FIG. 15 is a block diagram of the control device 50 of the X-ray inspection device 10 of the present embodiment. In the present embodiment, the X-ray inspection device 10 is further provided with a wireless communication device 70. The control device 50 is electrically connected to the wireless communication device 70. The control part 51 of the control device 50 further has a determination part 66.

### (1-1) Wireless communication device

The wireless communication device 70 performs wireless communication with a plurality of beacon terminals 80. The beacon terminals 80 are indicators (small portable instruments) held by users. Each of the users operates the X-ray inspection device 10 to perform the foreign substance contamination inspection on the food *A* while holding one beacon terminal 80. The beacon terminal 80 is placed in, for example, a pocket, etc., of the user's operational clothing. The wireless communication device 70 may optionally have an antenna for improving communication quality with the beacon terminals 80.

Each of the beacon terminals 80 utilizes power from an internal battery to transmit a signal of a prescribed length, referred to as a beacon, in a prescribed cycle. A beacon is a signal of an electric wave or of an infrared ray or another electromagnetic wave. Terminal identification information is included in the beacon. Terminal identification information is added to each beacon terminal 80 and is information for uniquely identifying the beacon terminal 80. The wireless communication device 70 receives the beacons transmitted from the plurality of beacon terminals 80. For that reason, a beacon terminal 80 that has transmitted a beacon received by the wireless communication device 70 can be specified from the terminal identification information included in that beacon.

### (1-2) Control device

The determination part 66 determines whether or not the user is present within a prescribed region. This "prescribed region" is hereinafter referred to as an authentication effective region. An authentication effective region is a region proximal to the X-ray inspection device 10. The proximal region is, for example, a region in which a distance to the X-ray inspection device 10 operated by the user is equal to or less than a prescribed length.

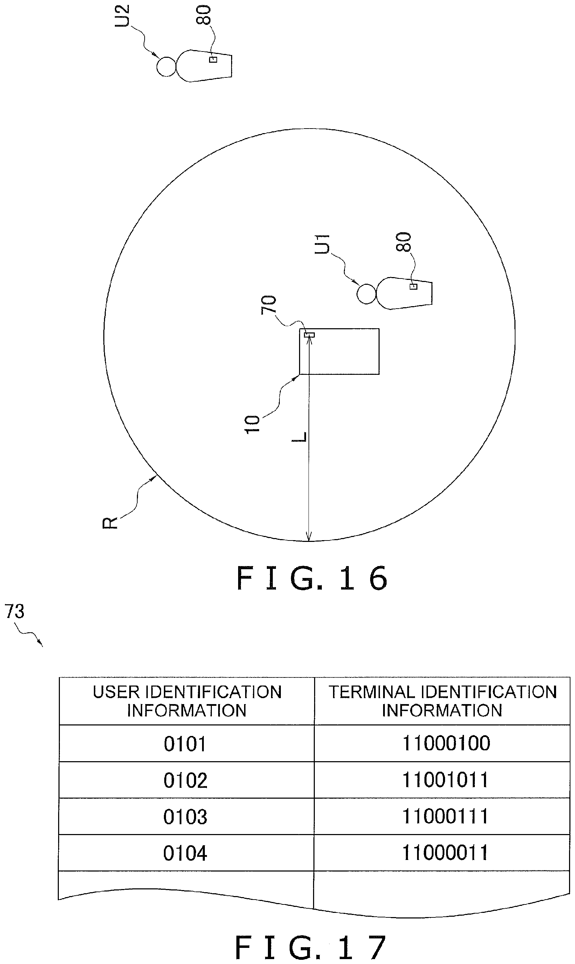

FIG. 16 is a drawing for describing an action of the determination part 66 and an authentication effective region *R.* The authentication effective region *R* shown in FIG. 16 is a circle centered around a position of the wireless communication device 70 of the X-ray inspection device 10, a radius of the circle being a prescribed length *L.* The prescribed length *L* is, for example, 5 meters. FIG. 16 shows two users U1, U2 holding beacon terminals 80. The user U1 is present inside the authentication effective region *R* and the user U2 is not present inside the authentication effective region *R.*

On the basis of a signal strength of a beacon that the wireless communication device 70 receives from a beacon terminal 80 held by a user, the determination part 66 is able to determine whether or not the user is present inside the authentication effective region *R.* As a distance between the wireless communication device 70 and the beacon terminal 80 lengthens, the signal strength of the beacon that the wireless communication device 70 receives from the beacon terminal 80 weakens commensurately. For that reason, when the signal strength of the beacon received by the wireless communication device 70 is equal to or less than a prescribed value, the determination part 66 senses that the beacon terminal 80 that transmitted the beacon is not present inside the authentication effective region *R* centered around the wireless communication device 70. Conversely, when the signal strength of the beacon received by the wireless communication device 70 is greater than the prescribed value, the determination part 66 senses that the beacon terminal 80 that transmitted the beacon is present inside the authentication effective region *R* centered around the wireless communication device 70. When the determination part 66 senses that the beacon terminal 80 is not present inside the authentication effective region *R,* the determination part 66 determines that the user holding the beacon terminal 80 is not present inside the authentication effective region *R.* Conversely, when the determination part 66 senses that the beacon terminal 80 is present inside the authentication effective region *R,* the determination part 66 determines that the user holding the beacon terminal 80 is present inside the authentication effective region *R.*

As described above, the beacon terminal 80 that transmitted the beacon received by the wireless communication device 70 can be specified from the terminal identification information included in that beacon. For that reason, the determination part 66 is able to sense the plurality of beacon terminals 80 distinctly from each other, and is therefore able to determine whether or not the respective users holding the beacon terminals 80 are present inside the authentication effective region *R.*

On the basis of the determination result of the determination part 66, the management part 64 automatically terminates the state of user authentication by the authentication part 63. Specifically, when the determination part 66 determines that the user is not present inside the authentication effective region *R,* the management part 64 automatically terminates the executable state of the sensitivity adjustment process performed by the processor 61.

With the X-ray inspection device 10, the user performs the authentication process on themselves on the basis of the data of the user's eye image captured using the iris-imaging device 60. For that reason, when the user is authenticated by the authentication part 63, the user is present in proximity to the iris-imaging device 60; i.e., inside the authentication effective region *R.* The authenticated user is able to use the X-ray inspection device 10 to perform the foreign substance contamination inspection on the food *A.* However, when the authenticated user has moved away from the X-ray inspection device 10 and left the authentication effective region *R,* the user is determined by the determination part 66 to not be present inside the authentication effective region *R* and the user authentication is automatically terminated by the management part 64.

The storage part 52 further has a beacon terminal information database 73. FIG. 17 is one example of the beacon terminal information database 73. The beacon terminal information database 73 correlates and stores users and the beacon terminals 80 held by the users. Specifically, the beacon terminal information database 73 is a database in which user identification information and terminal identification information of the beacon terminals 80 are correlated, and the user identification information is designated as a main key. The user identification information is an ID number shared with the user identification information used by the process information database 71. The terminal identification information is an ID number for uniquely identifying a beacon terminal 80. Each of the users holds only the beacon terminal 80 to which the terminal identification information correlated with the user's user identification information has been added.

The beacon terminal information database 73 is, for example, created in advance before the start of the handling of the X-ray inspection device 10 by the manager, etc., of the X-ray inspection device 10 or the inspection line 100, and is revised after the start of the handling of the X-ray inspection device 10.

The determination part 66 senses whether or not the beacon terminal 80 is present inside the authentication effective region *R,* and determines whether or not the user holding the beacon terminal 80 is present inside the authentication effective region *R.* In this determination process, the determination part 66 is able to reference the beacon terminal information database 73 to specify the user holding each beacon terminal 80.

### (2) Process of automatic termination of user authentication

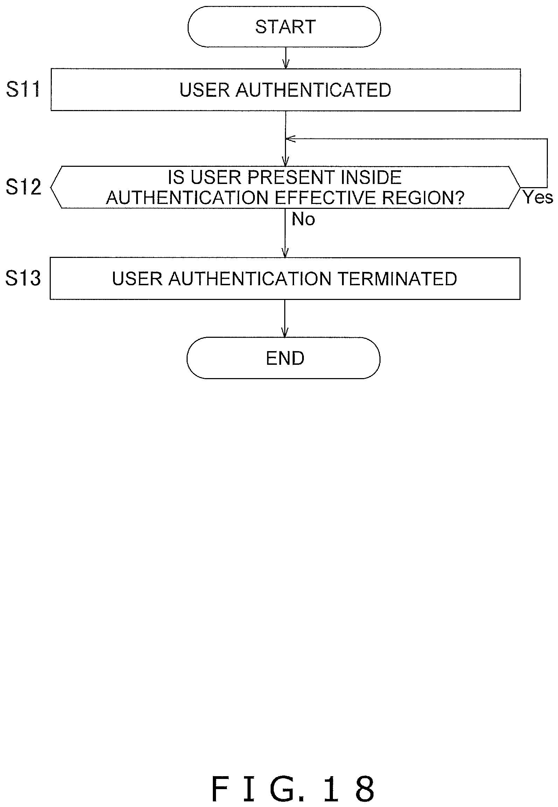

Next, a process by which the management part 64 automatically terminates the user authentication (automatic authentication termination process) shall be described. The automatic authentication termination process is executed while the authentication part 63 of the control device 50 of the X-ray inspection device 10 is authenticating at least one user. FIG. 18 is a flowchart representing a procedure of a user authentication process including the automatic authentication termination process.

In step S11, a user is authenticated through the authentication process shown in FIG. 9. The authenticated user is able to operate the X-ray inspection device 10 to perform the foreign substance contamination inspection on the food *A* in the authentication effective region *R* proximal to the X-ray inspection device 10.

In step S12, a determination is made as to whether or not the user is present inside the authentication effective region *R.* This determination process is performed by the determination part 66. If the user is determined to be present inside the authentication effective region *R,* the determination process in step S12 is executed again after a prescribed time has elapsed. If the user is determined to not be present inside the authentication effective region *R,* a transition is made to step S13.

In step S13, the authentication of the user authenticated in step S11 is terminated. The user whose authentication has been terminated cannot operate the X-ray inspection device 10 to perform the foreign substance contamination inspection on the food *A.* In this case, the user must perform the authentication process in step S11 again in order to perform the foreign substance contamination inspection on the food A again.

### (3) Characteristics

(3-1)
   The X-ray inspection device 10, which is a food-processing device according to the present embodiment, has a function for an automatic authentication termination process. Namely, when the user of the X-ray inspection device 10 moves a prescribed distance away from the X-ray inspection device 10 after the user has performed the authentication process and been authenticated, the user-authenticated state is automatically terminated. Namely, when the X-ray inspection device 10 can no longer be monitored because the authenticated user has moved away from the proximity of the X-ray inspection device 10, the user must perform the authentication process on themselves again in order to use the X-ray inspection device 10 to perform the foreign substance contamination inspection on the food *A.* Due to this configuration, it is possible with the X-ray inspection device 10 to suppress instances in which another user who has not been authenticated uses the X-ray inspection device 10 to perform the foreign substance contamination inspection on the food *A* while the authenticated user is not present in the proximity of the X-ray inspection device 10. Therefore, with the X-ray inspection device 10, usage of the device by users other than authenticated users can be suppressed.
(3-2)
   With the X-ray inspection device 10, a beacon terminal 80 held by a user can be utilized to ascertain, with high precision, whether or not the user is present in the proximity of the X-ray inspection device 10. Therefore, with the X-ray inspection device 10, the precision of the automatic authentication termination process can be improved by using the beacon terminals 80.
(3-3)
   With the X-ray inspection device 10, a plurality of beacon terminals 80 can be sensed distinctly from each other on the basis of the terminal identification information included in the beacons received by the wireless communication device 70 from the beacon terminals 80. For that reason, with the X-ray inspection device 10, each of the plurality of users holding the beacon terminals 80 can be determined to be present or not in the proximity of the X-ray inspection device 10.

Due to this configuration, when, for example, a certain authenticated user performs the foreign substance contamination inspection on the food *A* in the proximity of the X-ray inspection device 10 and this authenticated user is relieved from the inspection operation by another user who has not been authenticated, the authentication is automatically terminated when the authenticated user moves away from the X-ray inspection device 10, even if the unauthenticated user is in the proximity of the X-ray inspection device 10. For that reason, the user who has taken over the inspection operation from the previous user cannot perform the foreign substance contamination inspection on the food *A* as long as this user has not performed their own authentication process and been authenticated. Therefore, with the X-ray inspection device 10, it is possible to suppress instances in which users other than authenticated users use the device before being authenticated themselves.

### (4) Modifications

Although one embodiment of the present invention is described above, this embodiment is not provided by way of limitation on the present invention and various changes can be made within a range that does not deviate from the scope of the invention.

Modifications A to I of the first embodiment can also be applied to the present embodiment.

### (4-1) Modification A

In the embodiment, the automatic authentication termination process of the X-ray inspection device 10 is performed by the determination part 66 utilizing the beacon terminal 80, which is an indicator held by the user, to determine whether or not the user is present in the proximity of the X-ray inspection device 10. However, the automatic authentication termination process of the X-ray inspection device 10 may optionally be performed by utilizing a wireless communication terminal other than the beacon terminal 80 as the indicator held by the user. For example, the user may optionally hold a passive tag, which is a passive kind of IC tag, instead of the beacon terminal 80 as the indicator. The passive tag is, for example, an RF tag that utilizes RFID.

In this case, the passive tag held by the user uses electric waves transmitted from the wireless communication device 70 as an energy source and can reflect the received electric waves to return the reflected waves to the wireless communication device 70 with various kinds of information included. The passive tag includes its own identification information in a reflected wave signal, whereby the wireless communication device 70 is able to distinguish a plurality of passive tags from each other. Additionally, the determination part 66 can determine whether or not the user is present inside the authentication effective region R on the basis of a signal strength of the reflected waves received by the wireless communication device 70 from the passive tag held by the user. Namely, the passive tag can be utilized in the same method as the beacon terminal 80 of the embodiment.

The distance from the wireless communication device 70 at which the passive tag can be received is shorter than that of the beacon terminal 80. However, the passive tag is less costly than the beacon terminal 80 and can be mostly permanently activatable because an energy source is not required. The passive tag is also very lightweight and can therefore easily be attached to, for example, the user's operational clothing.

The X-ray inspection device 10 may optionally be configured such that the determination part 66 determines whether or not the user is present inside the authentication effective region *R* without utilizing a beacon terminal 80, a passive tag, or another indicator. For example, the determination part 66 may optionally determine whether or not the user is present inside the authentication effective region *R* due to an image or moving image of the X-ray inspection device 10 and the periphery thereof being captured in real time and the acquired image data or moving image data being analyzed. The determination part 66 may also optionally determine whether or not the user is present inside the authentication effective region *R* by utilizing laser sensing, etc.

### (4-2) Modification B

In the embodiment, the authentication of the authenticated user is terminated when the user is determined to not be present inside the authentication effective region *R,* as shown in FIG. 18. Namely, the management part 64 terminates the authentication of the user in the instant that the determination part 66 determines that the user is not present inside the authentication effective region *R.*

However, the management part 64 may optionally terminate the authentication of the user when the user continues to not be present inside the authentication effective region *R* for at least a prescribed time. Specifically, the determination part 66 determines that the user is not present inside the authentication effective region *R* when the user is sensed to not be present inside the authentication effective region *R* from a time point when the user is no longer inside the authentication effective region *R* until the elapse of a prescribed time (e.g., a short time of about one to three minutes). Due to this configuration, there is no need for the user to perform the authentication process again because the user authentication is not terminated even when, for example, the user temporarily leaves the authentication effective region *R* in order to operate the lighting in the room in which the X-ray inspection device 10 is installed and immediately returns to the same authentication effective region *R.*

### (4-3) Modification C

With the X-ray inspection device 10 of the embodiment, the plurality of beacon terminals 80 can be sensed distinctly from each other on the basis of the terminal identification information included in the beacons received by the wireless communication device 70 from the beacon terminals 80. However, with the X-ray inspection device 10, the plurality of beacon terminals 80 need not be sensed distinctly from each other. In this case, a user can hold any beacon terminal 80.

### (4-4) Modification D

In the embodiment, on the basis of the beacon terminal information database 73 created in advance by a manager, etc., of the X-ray inspection device 10 or the inspection line 100, the users hold beacon terminals 80 to which the terminal identification information correlated with their own user identification information has been added.

However, the users may, while holding any beacon terminal 80, optionally be correlated with the beacon terminal 80 they are holding when authenticated by the authentication part 63. In this case, the beacon terminal information database 73 is updated when a user is authenticated. Specifically, when a user is authenticated, the determination part 66 of the X-ray inspection device 10 specifies the beacon terminal 80 held by the user from the terminal identification information included in the beacon received by the wireless communication device 70 from the beacon terminal 80 held by the user. The control device 50 then correlates the user identification information of the authenticated user and the terminal identification information of the beacon terminal 80 held by the authenticated user, and registers the correlation in the beacon terminal information database 73.

### (4-5) Modification E

In the embodiment, after a user has been authenticated by the authentication part 63, the determination part 66 utilizes the beacon terminal 80 held by the user to determine whether or not the user is present inside the authentication effective region *R.* However, the authentication part 63 may optionally allow the user authentication process only when the determination part 66 has determined that the user is present inside the authentication effective region *R.*

### (4-6) Modification F

In the embodiment, the management part 64 of the X-ray inspection device 10 enables a user authenticated by the authentication part 63 to execute a process that is performed on the food *A* by the processor 61. In this modification, the X-ray inspection device 10 may optionally perform at least two kinds of processes on the food *A,* these processes including a first-type process and a second-type process. The first-type process is a process that is executable only by a user authenticated by the authentication part 63. The second-type process is a process that is executable by all users, including users authenticated by the authentication part 63 and users not authenticated by the authentication part 63. The first-type process is a process requiring authentication; e.g., the process of the foreign substance contamination inspection for the food *A.* The second-type process is a process not requiring authentication; e.g., a process of operating the monitor 40 to reference current settings. Information pertaining to the first-type process and the second-type process is stored in the storage part 52, and the management part 64 can determine if a process to be executed by a user is either the first-type process or the second-type process.

In the present modification, the X-ray inspection device 10 is able to distinguish between the first-type process requiring user authentication process and the second-type process not requiring user authentication process; therefore, there is no need to perform the authentication process for a user who is not performing a critical process (the first-type process) requiring special authority. Such users do not need to hold beacon terminals 80 and the number of beacon terminals 80 that need to be prepared can therefore be reduced.

### (4-7) Modification G

In the embodiment, the X-ray inspection device 10 utilizes iris information obtained from iris image data included in an image of the user's eye as biometric information for the user authentication process. However, the X-ray inspection device 10 may optionally utilize biometric information other than the iris for the user authentication process. For example, the X-ray inspection device 10 may optionally utilize a fingerprint, a voiceprint, a facial shape, etc., for the user authentication process.

The X-ray inspection device 10 may also optionally perform the user authentication process without utilizing biometric information. For example, the X-ray inspection device 10 may optionally perform the user authentication process using a password set by the user.

### <Third Embodiment>

### (1) Overall configuration of food-processing device management system

The food-processing device management system according to the present invention is a system for managing a plurality of food-processing devices. A food-processing device managed by the food-processing device management system of the present embodiment has a basic configuration and actions similar to those of the food-processing device according to the first embodiment or the second embodiment. The food-processing device is an X-ray inspection device 10 that uses X-rays to perform a foreign substance contamination inspection on a food A. As pertains to the X-ray inspection device 10, differences between the X-ray inspection device 10 of the present embodiment and the X-ray inspection device 10 of the first embodiment are described below.

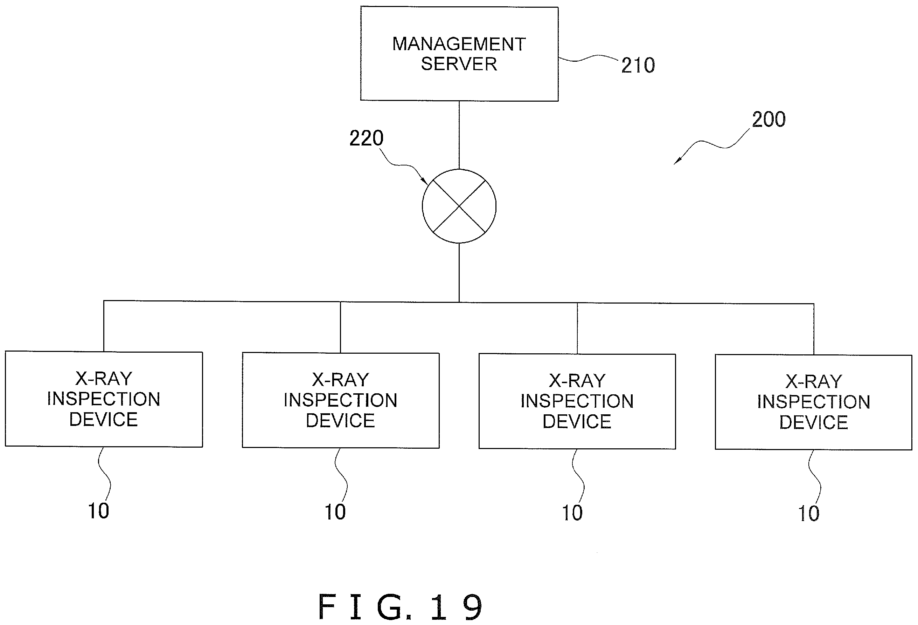

FIG. 19 is a diagram of the overall configuration of a food-processing device management system 200 of the present embodiment. The food-processing device management system 200 is primarily configured from a plurality of X-ray inspection devices 10 and one management server 210. The X-ray inspection devices 10 are food-processing devices managed by the food-processing device management system 200. The management server 210 is connected to the X-ray inspection devices 10 via a network 220.

### (2) Configuration of X-ray inspection devices

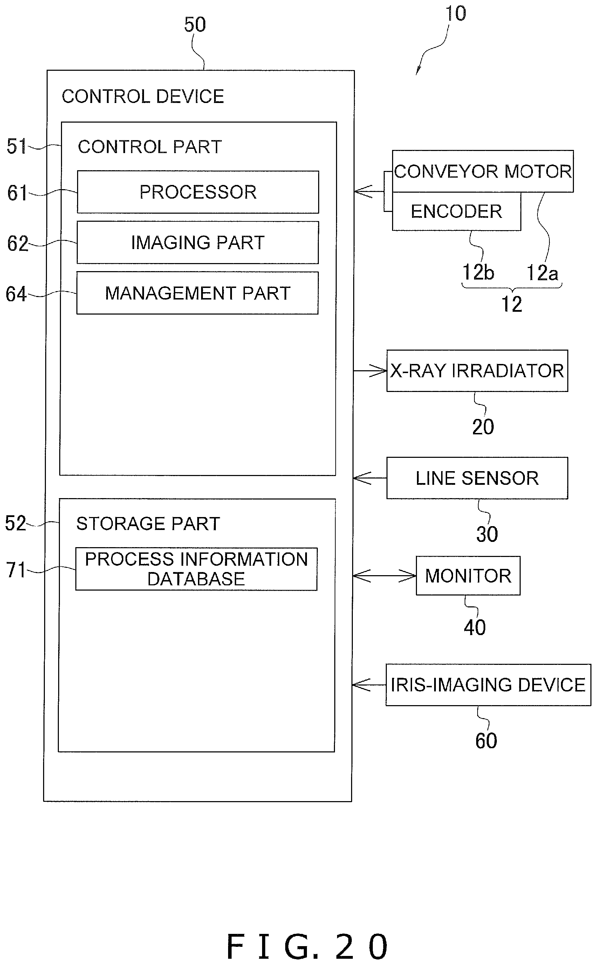

FIG. 20 is a block diagram of a control device 50 of an X-ray inspection device 10. Differences between the control device 50 of the present embodiment and the control device 50 of the first embodiment are described below.

### (2-1) Control part

A control part 51 primarily has a processor 61, an imaging part 62, and a management part 64. The control part 51 is primarily configured from a CPU, a ROM, and a RAM.

### (2-1-1) Imaging part

The control device 50 transmits a received image of a user's eye to the management server 210. The management server 210 uses the image of the user's eye received from the control device 50 to perform the user authentication process, and transmits the authentication result to the control device 50. Thus, the management server 210 authenticates users of each X-ray inspection device 10.

### (2-1-2) Management part

The management part 64 makes a process performed by the processor 61 executable for a user authenticated by the management server 210. Specifically, when the management server 210 authenticates a user, the management part 64 controls the X-ray inspection device 10 so that the authenticated user is able to start a sensitivity adjustment process performed by the processor 61. For example, when the management server 210 has authenticated a user, the management part 64 displays a screen image for executing the sensitivity adjustment process on the monitor 40.

### (2-2) Storage part

The storage part 52 is primarily configured from an HDD. The storage part 52 has a process information database 71. FIG. 7 is one example of the process information database 71. The process information database 71 correlates and stores users and processes made executable for the users by the management part 64. Specifically, the process information database 71 is a database in which the user identification information and process identification information are correlated, and the user identification information is set as a main key. The user identification information is an ID number for uniquely identifying the user. The process identification information is a bit string representing processes executable on the X-ray inspection device 10 by the user when the user has been authenticated by the management server 210. Each bit of the process identification information represents a corresponding "executable process."

The process information database 71 is, for example, created in advance before the start of the handling of the food-processing device management system 200 by a manager, etc., of the food-processing device management system 200, and is revised after the start of the handling of the system.

### (3) Configuration of management server

The management server 210 is a personal computer connected to each X-ray inspection device 10 via LAN cables, etc. The manager of the food-processing device management system 200 can operate the management server 210 to collectively manage the plurality of X-ray inspection devices 10.

The management server 210 is primarily configured from a CPU, a ROM, a RAM, a hard disk drive (HDD), etc. A solid state drive (SSD) may optionally be used instead of an HDD.

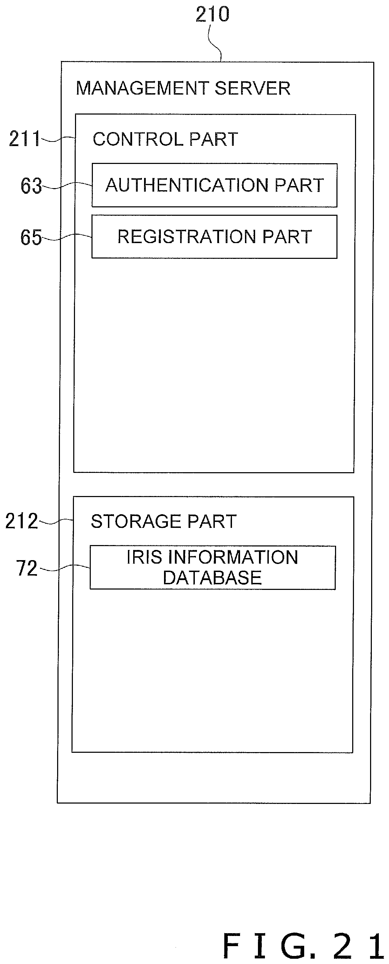

FIG. 21 is a block diagram of the management server 210. The management server 210 primarily has a control part 211 and a storage part 212.

The control part 211 is primarily configured from a CPU, a ROM, and a RAM. The control part 211 has an authentication part 63 and a registration part 65. These are each a function realized by executing a program stored in the storage part 212. The storage part 212 is primarily configured from an HDD. The storage part 212 has an iris information database 72.

### (3-1) Authentication part

The authentication part 63 performs the user authentication process on the basis of the data of the image of the user's eye transmitted from the control device 50 of the X-ray inspection device 10. The authentication part 63 sends the authentication result back to the control device 50 which transmitted the eye image used in the authentication process. The user authentication process is performed before the start of the use of the X-ray inspection device 10. The user is able to execute the sensitivity adjustment process of the X-ray inspection device 10 only when authenticated by the authentication part 63.

Specifically, the authentication part 63 first extracts a section corresponding to the user's iris from the image of the user's eye. Next, the authentication part 63 converts an image of only the section of the user's iris to iris information of the user, using a dedicated algorithm. The iris information is a bit string of a prescribed length which can be utilized as biometric authentication information.

Next, the authentication part 63 determines whether or not the iris information (during-authentication iris information) converted from the image of the user's eye before the start of the use of the X-ray inspection device 10 is included in iris information (registered iris information, described hereinafter) that has been registered in advance in the registration part 65. If the during-authentication iris information is included in the registered iris information, the authentication part 63 determines that the user has the authority to execute the sensitivity adjustment process of the X-ray inspection device 10. If the during-authentication iris information is not included in the registered iris information, the authentication part 63 determines that the user does not have the authority to execute the sensitivity adjustment process of the X-ray inspection device 10. An authenticated user is a user determined by the authentication part 63 to have the authority to execute the sensitivity adjustment process of the X-ray inspection device 10.

### (3-2) Registration part

The registration part 65 registers data pertaining to the image of the user's eye. Specifically, before the start of the use of the food-processing device management system 200, the registration part 65 registers iris information converted from the image of each user's eye for all users that could possibly use the X-ray inspection device 10. The registration part 65 registers user identification information and the registered iris information in correlation in the iris information database 72. The user identification information is an ID number for uniquely identifying a user. This ID number is, for example, a character string configured from a plurality of alphanumeric characters. The registered iris information is a bit string that is converted from the image of the user's eye captured in advance by the imaging part 62 of the X-ray inspection device 10, and that can be utilized as biometric authentication information.

### (3-3) Iris information database

FIG. 8 is one example of the iris information database 72. The iris information database 72 correlates and stores users and iris information (registered iris information) of the users registered in advance using the registration part 65. Specifically, the iris information database 72 is a database in which the user identification information and the registered iris information are correlated, and the user identification information is set as a main key. The user identification information is an ID number shared with the user identification information used by the process information database 71 of the X-ray inspection device 10. The registered iris information is a bit string that is converted from the image of the user's eye and that can be utilized as biometric authentication information.

The iris information database 72 is, for example, created in advance before the start of the handling of the food-processing device management system 200 by a manager, etc., of the food-processing device management system 200, and is revised after the start of the handling of the system.

A user can register their iris information in advance in the iris information database 72 of the management server 210 by using the iris-imaging device 60 of any X-ray inspection device 10 to capture an image of their eye. The iris information database 72 stores each user's iris information, which can be utilized in the authentication processes of all X-ray inspection devices 10. For that reason, the management server 210 can collectively manage the iris information of the users of all the X-ray inspection devices 10.

### (4) Authentication process of X-ray inspection device

FIG. 9 is a flowchart representing a procedure of the authentication process of the X-ray inspection device 10. Steps S1 and S2 pertain to the iris information registration process, which is performed in advance before the start of the authentication process of the X-ray inspection device 10. Steps S1 and S2 pertain to advance preparations and can be executed in any order. Steps S3 to S9 pertain to the user authentication process, which is performed before the start of the use of the X-ray inspection device 10.

In step S1, the user's iris information is registered. In step S1, each user of the X-ray inspection device 10 captures images of their eye using the iris-imaging device 60 of any X-ray inspection device 10, and registers their iris information in advance in the iris information database 72 of the management server 210. This operation is performed by all users who could possibly use the X-ray inspection device 10, before the sensitivity adjustment process of the X-ray inspection device 10 is started.

In step S2, information pertaining to processes executable on the X-ray inspection device 10 by users is registered in the process information databases 71 of the X-ray inspection devices 10 for the users registered in the iris information database 72 in step S1. This operation is normally performed by the manager of the food-processing device management system 200. The manager is able to operate the monitor 40 of each X-ray inspection device 10 or the management server 210, etc., input the information pertaining to the processes executable by each user, and register this information in the process information databases 71 of the X-ray inspection devices 10.

In step S3, preparation is performed for the user authentication process performed by the X-ray inspection device 10. This is normally performed immediately before a user starts to use the X-ray inspection device 10. The user is thereby able to execute the authentication process of the X-ray inspection device 10.

In step S4, the user captures an image of their eye using the iris-imaging device 60 of the X-ray inspection device 10. Specifically, the user removes the iris-imaging device 60 from the shield box 11 of the X-ray inspection device 10 and brings the protective cover 60e of the iris-imaging device 60 close to their eye. The imaging part 62 of the X-ray inspection device 10 thereby automatically captures an image of the user's eye using the iris-imaging device 60.

In step S5, the user's eye image captured in step S4 is transmitted to the management server 210, and the user's iris information is acquired by the authentication part 63 of the management server 210 on the basis of the transmitted eye image. The processes from step S5 onward are performed automatically.

In step S6, a determination is made by the authentication part 63 of the management server 210 as to whether or not the user's iris information acquired in step S5 is included in the iris information database 72 registered in step S1. If the determination result is `YES', the user is authenticated and a transition is made to step S7. If the determination result is 'NO', the user is not authenticated and a transition is made to step S9. In step S6, the management server 210 transmits the determination result (authentication result) to the X-ray inspection device 10 that transmitted the eye image in step S5. The processes in the subsequent steps S7 to S9 are performed by the management part 64 of the X-ray inspection device 10 that received the determination result from the management server 210.

In step S7, information pertaining to processes executable by the user authenticated in step S6 is acquired from the process information database 71 registered in step S2.

In step S8, the processes executable by the user, which were acquired in step S7, are put into a state of actually being executable on the X-ray inspection device 10 by the user. At this time, the user authenticated in step S6 and the processes acquired in step S7 and executable by the user may optionally be displayed on the monitor 40. The user is thereby able to refer to the monitor 40 to confirm the information pertaining to their authenticated self (name, department, etc.) and the processes executable by the user. From this point onward, the authenticated user is able to execute the sensitivity adjustment process of the X-ray inspection device 10.

In step S9, the user is given a report that, *inter alia,* the iris information of the user not authenticated in step S6 has not been registered in the iris information database 72. For example, the monitor 40 displays a warning message stating that, *inter alia,* "The device cannot be operated because the iris information has not been registered."

### (5) Characteristics

(5-1)
With the food-processing device management system 200 according to the present embodiment, users' iris information required for the authentication processes for the users of the X-ray inspection devices 10 can be collectively managed by the management server 210. Therefore, the food-processing device management system 200 can facilitate the management of biometric information used in the user authentication processes when managing a plurality of X-ray inspection devices 10 having the function of authenticating users.

Additionally, due to the food-processing device management system 200 collectively managing the information required for the user authentication processes, the order of the operations performed and the time taken for different kinds of operations become easily managed for each user, and data pertaining to subsequent examinations of operation specifics and to improving operational procedures can easily be acquired, for example. The food-processing device management system 200 is also able to easily check whether or not an incorrect authentication process has been performed. An incorrect authentication process is, for example, an authentication process in which the authenticated user and the user that performed the actual process after the authentication are suspected to not be the same person, such as an authentication process that the same user has performed simultaneously on a plurality of X-ray inspection devices 10 positioned apart from each other.
(5-2)
   In the food-processing device management system 200, when a user captures an eye image using the iris-imaging device 60 of any one of the X-ray inspection devices 10, the user's iris information acquired from the eye image is registered in the iris information database 72 of the management server 210. The management server 210 uses the iris information registered in the iris information database 72 to perform authentication for the user of the X-ray inspection device 10. Namely, once the user has registered iris information with any X-ray inspection device 10, the user is then able to perform the authentication process on all of the X-ray inspection devices 10. Therefore, the user need not register iris information in all of the X-ray inspection devices 10, and the food-processing device management system 200 can therefore easily register biometric information used in the user authentication process.
(5-3)
   In the food-processing device management system 200, each X-ray inspection device 10 has a process information database 71. The process information database 71 correlates and stores users and the processes that become executable for authenticated users. Therefore, the food-processing device management system 200 can manage the processes executable for the users for each X-ray inspection device 10.
(5-4)
   The X-ray inspection device 10 utilizes an image of a user's eye as biometric information for the user authentication process. Situations in which a user is authenticated who should not be, and situations in which the authenticated user is not the same person as the actual user, are thereby avoided. Consequently, only an authenticated user is able to execute a process with the X-ray inspection device 10.
(5-5)
   The X-ray inspection device 10 utilizes, as biometric information for the user authentication process, iris information obtained from the iris image data included in the image of the user's eye. Biometric authentication using the user's iris has a high identification capability similar to biometric authentication using the user's fingerprint, but is particularly suitable for the authentication process because of advantages such as the following. Firstly, the pattern of a human iris is complicated and is therefore suitable as information for specifying a user. Secondly, the pattern of a human iris mostly does not change even after a long period of time and is substantially impossible to falsify; therefore, iris information once registered can be utilized semi-permanently as highly reliable biometric information. Thirdly, non-contact authentication processes can be performed merely by capturing an image of the user's eye using a comparatively small device such as the iris-imaging device 60; therefore, there is no need to ensure a large space to install special instruments for the authentication process. Therefore, the X-ray inspection device 10 is capable of executing a highly precise and highly reliable authentication process.
(5-6)
   In a food-manufacturing plant, etc., in which the X-ray inspection device 10 is installed, users wear operational clothing that covers parts other than the eyes in order to prevent hair from becoming mixed into the food *A* and skin bacteria from adhering to the food *A.* In this case, of the parts of the user's body, only the user's eyes are exposed to the exterior. For that reason, information obtained from the image of the user's eye is used as biometric information capable of specifying the user, whereby the user is able to perform the authentication process and start using the X-ray inspection device 10 while wearing the operational clothing. Namely, the user need not remove the operational clothing to expose a part of the body in order to perform the authentication process; therefore, when the authentication process is performed, occurrences of problems in which the user's hair becomes mixed into the food *A* or bacteria of the user's skin adheres to the food *A* are suppressed. Therefore, the X-ray inspection device 10 is particularly useful in a food-manufacturing plant, etc., in which foreign substances must be prevented as much as possible from becoming mixed into and adhering to the food in order to guarantee product safety and quality.
(5-7)
   The storage part 52 of the X-ray inspection device 10 has the process information database 71, which correlates and stores users and processes that become executable for the users after authentication. For that reason, the manager (user having special authority) of the food-processing device management system 200 is able to operate the process information database 71 to collectively set and manage the processes executable by each user in advance. Situations in which, for example, a user executes critical operations that only a manager should execute are thereby avoided. Such critical operations are, for example, an operation adjusting the detection precision for foreign substances included in the food *A,* and such operations must be performed by a manager having the required skill and qualifications in order to guarantee safety and quality of the food *A.*

The critical operations that only a manager should execute are, for example, a process in a critical control point (CCP) of the food-manufacturing step, such as an operation adjusting the detection precision for foreign substances included in the food *A.* Such processes also include an operation adjusting a measurement precision for a temperature of the food *A,* an operation adjusting a measurement precision for a concentration of a harmful chemical substance adhering to the food *A,* etc. In the X-ray inspection device 10 in order to detect foreign substances included in the food *A* with high precision, for example, the foreign substance detection precision must be appropriately adjusted by means of a pre-adjustment or a periodic check performed by the manager. For that reason, in order to guarantee the safety and quality of the food *A,* the X-ray inspection device 10 is configured such that when an adjustment of the foreign substance detection precision is not performed in advance by a manager having special authority, a user not having special authority cannot start a foreign substance contamination inspection on the food *A.* Foreign substance detection errors in the foreign substance contamination inspection of the food *A* and other malfunctions resulting from a decrease in the foreign substance detection precision of the X-ray inspection device 10 are thereby suppressed.

(5-8)
The storage part 52 of the X-ray inspection device 10 has the iris information database 72, which correlates and stores users and iris information obtained from images of the users' eyes. The manager of the food-processing device management system 200 can thereby operate the iris information database 72 to easily perform the processes of registering and referencing the iris information for each user.

### (6) Modifications

Although one embodiment of the present invention is described above, this embodiment is not provided by way of limitation on the present invention and various changes can be made within a range that does not deviate from the scope of the invention.

Modifications A, C, E, F, H, and I of the first embodiment can also be applied to the present embodiment.

### (6-1) Modification A

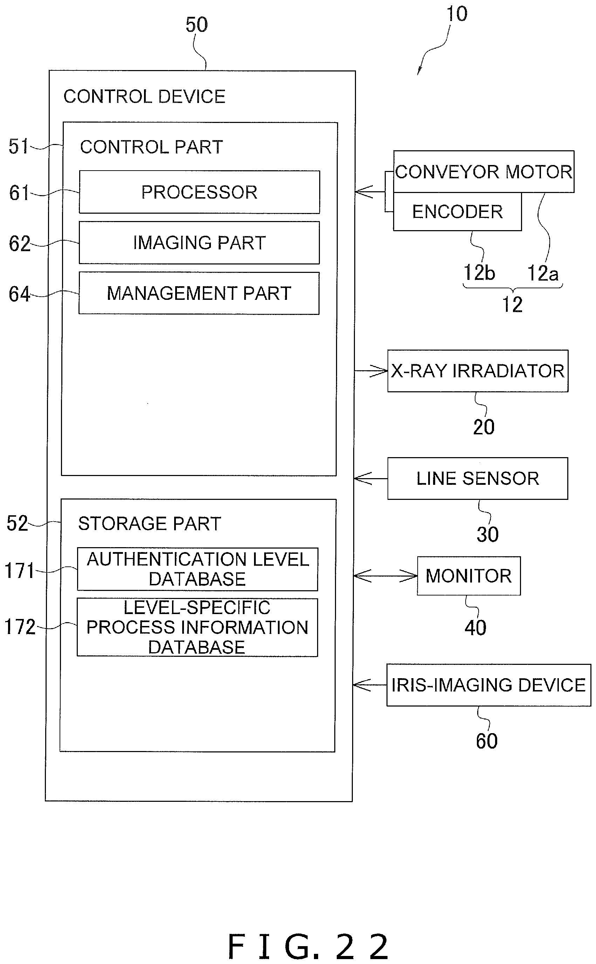

In the embodiment, the storage part 52 of the X-ray inspection device 10 has the process information database 71, which correlates and stores users and processes that become executable for the users after authentication. However, the storage part 52 of the X-ray inspection device 10 may optionally have, instead of the process information database 71, an authentication level database 171 and a level-specific process information database 172. FIG. 10 is one example of the authentication level database 171. FIG. 11 is one example of the level-specific process information database 172. FIG. 22 is a block diagram of the control device 50 in the present modification.

The authentication level database 171 correlates and stores users and authentication levels. In FIG. 10, user identification information serving as a main key and an authentication level are correlated for each user. The authentication level correlated with each user is only one of a plurality of authentication levels. In this modification, the lowest authentication level is 1 and the highest authentication level is 4. The authentication level represents the level of authority granted to the user.

For example, a user whose authentication level is 1 can execute only a process of the lowest limit required to determine the acceptability of the food A using the X-ray inspection device 10. A user whose authentication level is 2 is able to further execute a process controlling the action setting information such as the inspection speed of the food A, and the conveying direction of the conveying unit 12. A user whose authentication level is 3 is able to further execute a process in a critical control point (CCP) of a food-manufacturing step. In this modification, the process in the critical control point (CCP) of the food-manufacturing step is, for example, the sensitivity adjustment process of the embodiment or another process controlling inspection parameters affecting the foreign substance detection precision; an operation recording (periodic) measurement results of the temperature of the food A and an operation adjusting a measurement precision thereof; and an operation recording (periodic) measurement results of a concentration of a harmful chemical substance adhering to the food A and an operation adjusting a measurement precision thereof, etc. A user whose authentication level is 4 is able to further start up a special action mode of the X-ray inspection device 10. The special action mode is, for example, a mode for performing countermeasure and maintenance checkup operations during an emergency of the X-ray inspection device 10, and a mode for performing an operation registering a user having special authority when delivering the X-ray inspection device 10. In the present modification, a user granted any of the authentication levels 1 to 4 must perform an authentication process using iris information included in an image of their eye in order to perform processes pertaining to the manufacture of the food *A* using the X-ray inspection device 10.

The level-specific process information database 172 correlates and stores authentication levels and processes that become executable upon authentication. In FIG. 11, an authentication level serving as a main key and process identification information are correlated for each authentication level. The process identification information, as in the embodiment, is a bit string representing processes that become executable upon authentication.

In the present modification, the authentication part 63 acquires information pertaining to a process executable by the user authenticated in step S6, this information being acquired in step S7 in FIG. 9 of the embodiment on the basis of the authentication level database 171 and the level-specific process information database 172. Specifically, the authentication part 63 first acquires the authentication level of the authenticated user on the basis of the authentication level database 171. Next, the authentication part 63 acquires, on the basis of the level-specific process information database 172, information corresponding to the authentication level of the authenticated user and pertaining to the process executable by the user.

In the present modification, the manager of the food-processing device management system 200 is able to sort users into a plurality of groups by authentication level and set processes executable by the users for each group. Namely, the manager need not set processes executable by the user for each user. For that reason, with the X-ray inspection device 10 of the present modification, processes executable by the users can be easily managed by setting the executable processes in advance for each authentication level and setting an authentication level for each user.

### (6-2) Modification B

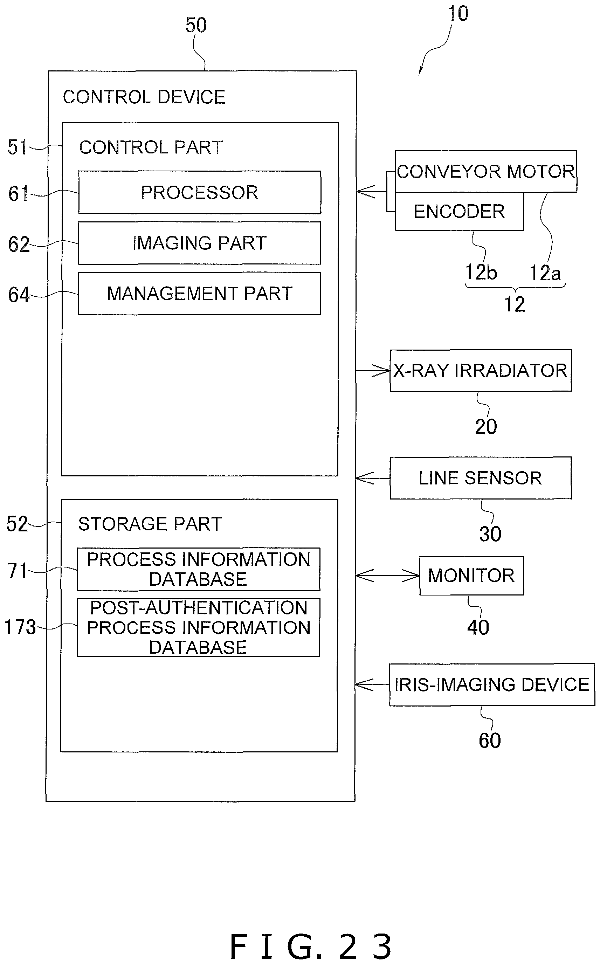

The storage part 52 of the X-ray inspection device 10 may also optionally be provided with a post-authentication process information database 173. FIG. 13 is one example of the post-authentication process information database 173. FIG. 23 is a block diagram of the control device 50 in the present modification. The post-authentication process information database 173 correlates and stores users authenticated by the authentication part 63 and processes made executable for the users by the management part 64. Specifically, the post-authentication process information database 173 correlates user identification information serving as a main key and post-authentication process identification information for each user. The post-authentication process identification information represents processes that become executable for the user after the user has been authenticated. The post-authentication process identification information, as with the process identification information of the embodiment, is a bit string representing processes performed by the processor 61.

In the present modification, the X-ray inspection device 10 stores authenticated users and post-authentication operation specifics of the users in correlation in the post-authentication process information database 173 in advance, whereby the traceability of the food A can be improved. The operation specifics include not only information representing executed processes (process IDs, etc.), but also data (values) acquired during the processes, the results of the processes, etc.

In the present modification, the control device 50 preferably prohibits changes and deletions of the record included in the post-authentication process information database 173. Namely, the post-authentication process information database 173 is preferably configured so that additions to the record are allowed but changes and deletions of the record are prohibited. In this case, falsification of the post-authentication process information database 173 is prevented and the reliability of the information stored in the post-authentication process information database 173 is therefore improved. The control device 50 may optionally be configured so that the record can be deleted upon fulfilment of conditions by which record preservation is guaranteed, in cases such as when the transfer of the record to another recording medium is complete.

As an option in the present modification, the storage part 212 of the management server 210, rather than the storage part 52 of the X-ray inspection device 10, may store the post-authentication process information database 173. In this case, the post-authentication process information database 173 correlates and stores users authenticated by the authentication part 63 and processes made executable for the users by the management part 64, for each X-ray inspection device 10 connected to the management server 210. The food-processing device management system 200 can thereby collectively manage authenticated users and post-authentication operation specifics of the users in correlation across a plurality of X-ray inspection devices 10.

### (6-3) Modification C

In the embodiment, the management part 64 of the X-ray inspection device 10 makes a process performed by the processor 61 executable for a user authenticated by the authentication part 63. However, when a prescribed time has elapsed after the user was authenticated by the authentication part 63, the management part 64 may optionally render the process performed by the processor 61 unexecutable until the user has again been authenticated by the authentication part 63. The prescribed time may optionally be set to be a fixed length of time, the time required for the process performed by the processor 61, or a time such as the time for which the X-ray inspection device 10 remained unoperated. In this case, even when the authentication process is performed and the foreign substance contamination inspection becomes executable, if the prescribed time has elapsed since the authentication, the user cannot proceed with the foreign substance contamination inspection without performing another authentication process. Due to this configuration, occurrences of the malfunction of another user being able to execute the foreign substance contamination inspection without performing the authentication process based on an image of their eye while the user authenticated by the authentication process is temporarily away from the X-ray inspection device 10 are suppressed. Namely, in the present modification, the X-ray inspection device 10 is capable of suppressing instances in which an actual user continues to be able to execute the process when the authenticated user is a different person from the actual user.

Additionally, when, for example, the manager of the food-processing device management system 200 performs an operation adjusting the foreign substance detection precision after being authenticated and another adjustment operation is performed after the first adjustment operation has been completed, it is sometimes unclear whether or not the second and subsequent instances of the adjustment operation were performed by the authenticated manager. For that reason, in the present modification, when a user authenticated by the authentication part 63 has performed a specific process (e.g., an operation adjusting the foreign substance detection precision), the management part 64 may optionally render this specific process performed by the processor 61 unexecutable until this user is again authenticated by the authentication part 63. Due to this configuration, in the example described above, the manager of the food-processing device management system 200 must perform the authentication process every time the operation adjusting the foreign substance detection precision is performed, and instances in which the adjustment operation is performed by a user not having special authority are therefore avoided.

### (6-4) Modification D

In Modification D, each X-ray inspection device 10 is provided with the process information database 71 (a second storage part) and the post-authentication process information database 173 (a third storage part). However, the management server 210 may optionally be provided with one or both of the process information database 71 and the post-authentication process information database 173.

### (6-5) Modification E

In the embodiment, the storage part 52 of each X-ray inspection device 10 in the food-processing device management system 200 has a process information database 71. However, as an option, the storage part 212 of the management server 210, rather than the storage part 52 of the X-ray inspection device 10, may have the process information database 71 of each X-ray inspection device 10.

In Modification B, the storage parts 52 of the X-ray inspection devices 10 have an authentication level database 171 and a level-specific process information database 172 instead of the process information database 71. However, as an option, the storage part 212 of the management server 210, rather than the storage parts 52 of the X-ray inspection devices 10, may have the authentication level databases 171 and the level-specific process information databases 172 of the X-ray inspection devices 10.

In the present modification, the food-processing device management system 200 collectively manages users and processes that authenticated users can execute in each X-ray inspection device 10 in correlation across a plurality of X-ray inspection devices 10.

### INDUSTRIAL APPLICABILITY

The food-processing device according to the present invention is useful for guaranteeing safety and quality of a food because only an authenticated user is able to execute a process.

### REFERENCE SIGNS LIST

- 10: X-ray inspection device (food-processing device)
- 60: Iris-imaging device (imaging device)
- 61: Processor
- 62: Imaging part (biometric information acquisition part)
- 63: Authentication part
- 64: Management part
- 66: Determination part
- 71: Process information database (second storage part)
- 72: Iris information database (fourth storage part)
- 73: Beacon terminal information database (first storage part)
- 80: Beacon terminal (indicator)
- 173: Post-authentication process information database (third storage part)
- 200: Food-processing device management system
- A: Food
- R: Authentication effective region (prescribed region)

### CITATION LIST

### PATENT LITERATURE

Patent Literature 1: Japanese Laid-open Patent Publication No. 2014-71072

## Claims

1. A food-processing device configured to process a food in a line that manufactures the food, the food-processing device comprising:
an X-ray inspection device (10),
a processor (61) configured to perform, using the X-ray inspection device (10), at least a sensitivity adjustment process for adjusting sensitivity for determining acceptability of the food;
an imaging device configured to capture an image of an eye of a user of the food-processing device;
an authentication part (63) configured to authenticate the user on the basis of the image of the user's eye captured by the imaging device; and
a management part (64) configured to make the process performed by the processor executable for the user authenticated by the authentication part,
**characterized in that**
the authentication part is configured to authenticate the user on the basis of information of the user's iris included in the image of the user's eye,
only when the authentication part (63) authenticates the user, the management part (64) is configured to control the X-ray inspection device (10) so that the authenticated user is able to start the sensitivity adjustment process performed by the processor (61).

2. The food-processing device according to claim 1, wherein:
the food-processing device further comprises a determination part configured to determine whether or not the user is present inside a prescribed region; and
the management part is configured to automatically terminate the executable state of the process performed by the processor on the basis of a determination result from the determination part.

3. The food-processing device according to claim 2, wherein:
the determination part is configured to determine whether or not the user is present inside the prescribed region proximal to the food-processing device; and
the management part is configured to automatically terminate the executable state of the process performed by the processor when the determination part determines that the user is not present inside the prescribed region.

4. The food-processing device according to claim 3, wherein:
the determination part is configured to sense whether or not an indicator held by the user is present inside the prescribed region to determine whether or not the user is present inside the prescribed region.

5. The food-processing device according to claim 4, wherein:
the food-processing device further comprises a first storage part that correlates and stores the user and the indicator;
the determination part can sense a plurality of indicators distinctly from each other; and
the determination part is configured to determine that the user is not present inside the prescribed region upon sensing that the indicator which is stored in the first storage part and which corresponds to the user authenticated by the authentication part is not present inside the prescribed region.

6. The food-processing device according to any one of claims 1 to 5, wherein the food-processing device further comprises:
a second storage part that correlates and stores the user and the process made executable for the user by the management part.

7. The food-processing device according to claim 6, wherein:
the second storage part correlates and stores
an authentication level which is one of a plurality of authentication levels and which is set for each user, and
the process made executable by the management part, said process being set for each authentication level.

8. The food-processing device according to claim 7, wherein:
the authentication part is configured to further authenticate, on the basis of information other than an image of the user's eye, users for whom the highest authentication level among the plurality of authentication levels has been set.

9. The food-processing device according to any one of claims 1 to 8, wherein:
the processor is configured to perform at least two kinds of processes including a first-type process and a second-type process on the food; and
the management part is configured to make the first-type process performed by the processor executable for only the user authenticated by the authentication part and make the second-type process performed by the processor executable for all the users.

10. The food-processing device according to any one of claims 1 to 9, wherein the food-processing device further comprises:
a third storage part that correlates and stores the user authenticated by the authentication part and the process made executable for the user by the management part.

11. The food-processing device according to claim 10, wherein:
the third storage part is configured so as not to accept changes or deletions to the correlated and stored user authenticated by the authentication part and the correlated and stored process made executable for the user by the management part.

12. The food-processing device according to any one of claims 1 to 11, wherein the food-processing device further comprises:
a fourth storage part that correlates and stores the image of the user's eye captured by the imaging device and user identification information for uniquely identifying the user.

13. The food-processing device according to any one of claims 1 to 12, wherein:
the imaging device is configured such that at least one of a position and an orientation of the imaging device is adjustable in accordance with a position of the user's eye.

14. The food-processing device according to any one of claims 1 to 13, wherein:
when a prescribed time has elapsed after the user was authenticated by the authentication part, the management part renders the process performed by the processor unexecutable until the user is again authenticated by the authentication part.

15. The food-processing device according to any one of claims 1 to 14, wherein:
the processor is configured to perform the process of determining the presence/absence of foreign substances included in the food.

16. A food-processing device management system (200) configured to manage a plurality of food-processing devices that process a food in a line in which the food is manufactured, the food-processing device management system (200) comprising:
the food-processing devices each of which has an X-ray inspection device (10), the X-ray inspection device (10) being configured to perform a sensitivity adjustment process, using the X-ray inspection device (10), for adjusting sensitivity for determining acceptability of the food,
a biometric information acquisition part (60) configured to acquire biometric information of users of the food-processing devices;
one first storage part (72) that correlates and stores, for each user, the biometric information and user identification information for uniquely identifying the user;
a second storage part (71) that stores executability information pertaining to a process the users can execute using the food-processing devices;
an authentication part (63) configured to authenticate the users on the basis of the biometric information acquired by the biometric information acquisition part and the biometric information stored in the first storage part (72); and
a management part (64) configured to, on the basis of the executability information stored in the second storage part (71), make the process performed by the food-processing devices executable for the users authenticated by the authentication part (63),
**characterized in that**
the biometric information is information of the user's iris included in the image of the user's eye,
only when the authentication part (63) authenticates the user, the management part (64) is configured to control the X-ray inspection device (10) so that the authenticated user is able to start the sensitivity adjustment process performed by the X-ray inspection device (10).

17. The food-processing device management system according to claim 16, further comprising:
a plurality of the food-processing devices.

18. The food-processing device management system according to claim 17, wherein:
the food-processing devices are configured to perform the process of determining the presence/absence of foreign substances included in the food.

19. The food-processing device management system according to claim 17 or 18, wherein:
each of the plurality of food-processing devices has at least one biometric information acquisition part; and
the first storage part correlates and stores the user identification information and the biometric information acquired by any of the biometric information acquisition parts.

20. The food-processing device management system according to any one of claims 17 to 19, wherein:
each of the plurality of food-processing devices has the second storage part.

21. The food-processing device management system according to any one of claims 16 to 20, further comprising:
a third storage part that correlates and stores the user authenticated by the authentication part and the process made executable for the user by the management part.

22. The food-processing device management system according to claim 21, wherein:
the third storage part is configured so as not to accept changes or deletions to the correlated and stored user authenticated by the authentication part and the correlated and stored process made executable for the user by the management part.

23. The food-processing device management system according to claim 21 or 22, wherein:
the third storage part correlates and stores, for each of the food-processing devices, the user authenticated by the authentication part and the process made executable for the user by the management part.

24. The food-processing device management system according to any one of claims 16 to 23, wherein:
the second storage part correlates and stores the user identification information and the executability information.

25. A food-processing device management method for managing a plurality of food-processing devices that process a food, the food-processing device management method comprising:
performing a sensitivity adjustment process, using an X-ray inspection device (10) of any one of the food-processing devices, for adjusting sensitivity for determining acceptability of the food,
acquiring biometric information of users of the food-processing devices;
correlating and storing, for each user, the biometric information and user identification information for uniquely identifying the user;
storing executability information pertaining to a process that is executable for the user by using the food-processing device;
authenticating the user on the basis of the acquired biometric information and the biometric information stored in correlation with the user identification information; and
making the process performed by the food-processing device executable for the authenticated user on the basis of the stored executability information;
**characterized in that**
the biometric information is information of the user's iris included in an image of the user's eye,
only when the authentication part (63) authenticates the user, controlling the X-ray inspection device (10) by the management part (64) so that the authenticated user is able to start the sensitivity adjustment process performed by the X-ray inspection device (10).

## Patentansprüche

1. Nahrungsmittelverarbeitungsvorrichtung, die konfiguriert ist, ein Nahrungsmittel in einer Linie zu verarbeiten, die das Nahrungsmittel herstellt, wobei die Nahrungsmittelverarbeitungsvorrichtung aufweist:
eine Röntgenprüfvorrichtung (10),
einen Prozessor (61), der konfiguriert ist, unter Verwendung der Röntgenprüfvorrichtung (10) mindestens einen Empfindlichkeitseinstellungsprozess zum Einstellen der Empfindlichkeit zur Feststellung der Annehmbarkeit des Nahrungsmittels durchzuführen;
eine Abbildungsvorrichtung, die konfiguriert ist, ein Bild eines Auges eines Benutzers der
Nahrungsmittelverarbeitungsvorrichtung zu erfassen;
einen Authentifizierungsteil (63), der konfiguriert ist, den Benutzer auf der Grundlage des Bildes des Auges des Benutzers, das von der Abbildungsvorrichtung erfasst wurde, zu authentifizieren; und
einen Verwaltungsteil (64), der konfiguriert ist, den vom Prozessor durchgeführten Prozess für den von dem Authentifizierungsteil authentifizierten Benutzer ausführbar zu machen,
**dadurch gekennzeichnet, dass**
der Authentifizierungsteil konfiguriert ist, den Benutzer auf der Grundlage von Informationen über die Iris des Benutzers zu authentifizieren, die im Bild des Auges des Benutzers enthalten sind,
nur wenn der Authentifizierungsteil (63) den Benutzer authentifiziert, der Verwaltungsteil (64) konfiguriert ist, die Röntgenprüfvorrichtung (10) so zu steuern, dass der authentifizierte Benutzer in der Lage ist, den vom Prozessor (61) durchgeführten Empfindlichkeitseinstellungsprozess zu starten.

2. Nahrungsmittelverarbeitungsvorrichtung nach Anspruch 1, wobei:
die Nahrungsmittelverarbeitungsvorrichtung ferner einen Feststellungsteil aufweist, der konfiguriert ist festzustellen, ob sich der Benutzer innerhalb eines vorgeschriebenen Bereichs befindet oder nicht; und
der Verwaltungsteil konfiguriert ist, den ausführbaren Zustand des vom Prozessor durchgeführten Prozesses auf der Grundlage eines Feststellungsergebnisses vom Feststellungsteil automatisch zu beenden.

3. Nahrungsmittelverarbeitungsvorrichtung nach Anspruch 2, wobei:
der Feststellungsteil konfiguriert ist festzustellen, ob sich der Benutzer innerhalb des vorgeschriebenen Bereichs in der Nähe der Nahrungsmittelverarbeitungsvorrichtung befindet oder nicht; und
der Verwaltungsteil konfiguriert ist, den ausführbaren Zustand des vom Prozessor durchgeführten Prozesses automatisch zu beenden, wenn der Feststellungsteil feststellt, dass sich der Benutzer nicht innerhalb des vorgeschriebenen Bereichs befindet.

4. Nahrungsmittelverarbeitungsvorrichtung nach Anspruch 3, wobei:
der Feststellungsteil konfiguriert ist zu erfassen, ob sich ein vom Benutzer gehaltener Indikator innerhalb des vorgeschriebenen Bereichs befindet oder nicht, um festzustellen, ob sich der Benutzer innerhalb des vorgeschriebenen Bereichs befindet oder nicht.

5. Nahrungsmittelverarbeitungsvorrichtung nach Anspruch 4, wobei:
die Nahrungsmittelverarbeitungsvorrichtung ferner einen ersten Speicherteil aufweist, der den Benutzer und den Indikator korreliert und speichert;
der Feststellungsteil mehrere Indikatoren getrennt voneinander erfassen kann; und
der Feststellungsteil konfiguriert ist festzustellen, dass sich der Benutzer nicht innerhalb des vorgeschriebenen Bereichs befindet, wenn er feststellt, dass der Indikator, der im ersten Speicherteil gespeichert ist und der dem durch den Authentifizierungsteil authentifizierten Benutzer entspricht, sich nicht innerhalb des vorgeschriebenen Bereichs befindet.

6. Nahrungsmittelverarbeitungsvorrichtung nach einem der Ansprüche 1 bis 5, wobei die Nahrungsmittelverarbeitungsvorrichtung ferner aufweist:
einen zweiten Speicherteil, der den Benutzer und den Prozess, der für den Benutzer durch den Verwaltungsteil ausführbar gemacht wird, korreliert und speichert.

7. Nahrungsmittelverarbeitungsvorrichtung nach Anspruch 6, wobei:
der zweite Speicherteil korreliert und speichert:
eine Authentifizierungsstufe, die eine aus mehreren Authentifizierungsstufen ist und für jeden Benutzer eingestellt wird, und
den Prozess, der durch den Verwaltungsteil ausführbar gemacht wird, wobei der Prozess für jede Authentifizierungsstufe eingestellt wird.

8. Nahrungsmittelverarbeitungsvorrichtung nach Anspruch 7, wobei:
der Authentifizierungsteil konfiguriert ist, ferner, auf der Grundlage von anderen Informationen als einem Bild des Auges des Benutzers, Benutzer zu authentifizieren, für die die höchste Authentifizierungsstufe aus den mehreren Authentifizierungsstufen eingestellt worden ist.

9. Nahrungsmittelverarbeitungsvorrichtung nach einem der Ansprüche 1 bis 8, wobei:
der Prozessor konfiguriert ist, mindestens zwei Arten von Prozessen, die einen Prozess erster Art und einen Prozess zweiter Art umfassen, am Nahrungsmittel durchzuführen; und
der Verwaltungsteil konfiguriert ist, den vom Prozessor durchgeführten Prozess der ersten Art nur für den durch den Authentifizierungsteil authentifizierten Benutzer ausführbar zu machen und den vom Prozessor durchgeführten Prozess der zweiten Art für alle Benutzer ausführbar zu machen.

10. Nahrungsmittelverarbeitungsvorrichtung nach einem der Ansprüche 1 bis 9, wobei die Nahrungsmittelverarbeitungsvorrichtung ferner aufweist:
einen dritten Speicherteil, der den durch den Authentifizierungsteil authentifizierten Benutzer und den durch den Verwaltungsteil für den Benutzer ausführbar gemachten Prozess korreliert und speichert.

11. Nahrungsmittelverarbeitungsvorrichtung nach Anspruch 10, wobei:
der dritte Speicherteil konfiguriert ist, keine Änderungen oder Löschungen am korrelierten und gespeicherten Benutzer, der durch den Authentifizierungsteil authentifiziert wird, und am korrelierten und gespeicherten Prozess, der für den Benutzer durch den Verwaltungsteil ausführbar gemacht wird, anzunehmen.

12. Nahrungsmittelverarbeitungsvorrichtung nach einem der Ansprüche 1 bis 11, wobei die Nahrungsmittelverarbeitungsvorrichtung ferner aufweist:
einen vierten Speicherteil, der das von der Abbildungsvorrichtung erfasste Bild des Auges des Benutzers und Benutzeridentifikationsinformationen zum eindeutigen Identifizieren des Benutzers korreliert und speichert.

13. Nahrungsmittelverarbeitungsvorrichtung nach einem der Ansprüche 1 bis 12, wobei:
die Abbildungsvorrichtung konfiguriert ist, mindestens eine Position und eine Ausrichtung der Abbildungsvorrichtung gemäß einer Position des Auges des Benutzers einstellbar ist.

14. Nahrungsmittelverarbeitungsvorrichtung nach einem der Ansprüche 1 bis 13, wobei:
wenn eine vorgeschriebene Zeit verstrichen ist, nachdem der Benutzer durch den Authentifizierungsteil authentifiziert wurde, der Verwaltungsteil den vom Prozessor durchgeführten Prozess nicht ausführbar macht, bis der Benutzer erneut durch den Authentifizierungsteil authentifiziert wird.

15. Nahrungsmittelverarbeitungsvorrichtung nach einem der Ansprüche 1 bis 14, wobei:
der Prozessor konfiguriert ist, den Prozess der Feststellung des Vorhandenseins/der Abwesenheit von in den Nahrungsmitteln enthaltenen Fremdstoffen durchzuführen.

16. Nahrungsmittelverarbeitungsvorrichtungs-Verwaltungssystem (200), das konfiguriert ist, mehrere Nahrungsmittelverarbeitungsvorrichtungen zu verwalten, die ein Nahrungsmittel in einer Linie verarbeiten, in der das Nahrungsmittel hergestellt wird, wobei das Nahrungsmittelverarbeitungsvorrichtungs-Verwaltungssystem (200) aufweist:
die Nahrungsmittelverarbeitungsvorrichtungen, von denen jede eine Röntgenprüfvorrichtung (10) aufweist, wobei die Röntgenprüfvorrichtung (10) konfiguriert ist, unter Verwendung der Röntgenprüfvorrichtung (10) einen Empfindlichkeitseinstellungsprozess durchzuführen, um die Empfindlichkeit zur Feststellung der Annehmbarkeit des Nahrungsmittels einzustellen,
einen Erfassungsteil (60) biometrischer Informationen, der konfiguriert ist, biometrische Informationen von Benutzern der Nahrungsmittelverarbeitungsvorrichtungen zu erfassen;
einen ersten Speicherteil (72), der für jeden Benutzer die biometrischen Informationen und
Benutzeridentifikationsinformationen zum eindeutigen Identifizieren des Benutzers korreliert und speichert; einen zweiten Speicherteil (71), der
Ausführbarkeitsinformationen speichert, die sich auf einen Prozess beziehen, den die Benutzer unter Verwendung der Nahrungsmittelverarbeitungsvorrichtungen ausführen können;
einen Authentifizierungsteil (63), der konfiguriert ist, die Benutzer auf der Grundlage der biometrischen Informationen, die durch den Erfassungsteil biometrischer Informationen erfasst werden, und der biometrischen Informationen, die in dem ersten Speicherteil (72) gespeichert sind, zu authentifizieren; und
einen Verwaltungsteil (64), der konfiguriert ist, auf der Grundlage der im zweiten Speicherteil (71) gespeicherten Ausführbarkeitsinformationen den von den Nahrungsmittelverarbeitungsvorrichtungen durchgeführten Prozess für die vom Authentifizierungsteil (63) authentifizierten Benutzer ausführbar zu machen,
**dadurch gekennzeichnet, dass**
die biometrischen Informationen Informationen über die Iris des Benutzers sind, die im Bild des Auges des Benutzers enthalten sind,
nur dann, wenn der Authentifizierungsteil (63) den Benutzer authentifiziert, der Verwaltungsteil (64) konfiguriert ist, die Röntgenprüfvorrichtung (10) so zu steuern, dass der authentifizierte Benutzer in der Lage ist, den von der Röntgenprüfvorrichtung (10) durchgeführten Empfindlichkeitseinstellungsprozess zu starten.

17. Nahrungsmittelverarbeitungsvorrichtungs-Verwaltungssystem nach Anspruch 16, das ferner aufweist:
mehrere Nahrungsmittelverarbeitungsvorrichtungen.

18. Nahrungsmittelbearbeitungsvorrichtungs-Verwaltungssystem nach Anspruch 17, wobei:
die Nahrungsmittelverarbeitungsvorrichtungen konfiguriert sind, den Prozess der Feststellung des
Vorhandenseins/Fehlens von Fremdstoffen in den Nahrungsmitteln durchzuführen.

19. Nahrungsmittelbearbeitungsvorrichtungs-Verwaltungssystem nach Anspruch 17 oder 18, wobei:
jede der mehreren Nahrungsmittelverarbeitungsvorrichtungen mindestens einen Erfassungsteil biometrischer Informationen Teil aufweist; und
der erste Speicherteil die Benutzeridentifikationsinformationen und die biometrischen Informationen, die von jedem der Erfassungsteile biometrischer Informationen erfasst wurden, korreliert und speichert.

20. Nahrungsmittelverarbeitungsvorrichtungs-Verwaltungssystem nach einem der Ansprüche 17 bis 19, wobei:
jede der mehreren Nahrungsmittelverarbeitungsvorrichtungen den zweiten Speicherteil aufweist.

21. Nahrungsmittelverarbeitungsvorrichtungs-Verwaltungssystem nach einem der Ansprüche 16 bis 20, das ferner aufweist: einen dritten Speicherteil, der den durch den Authentifizierungsteil authentifizierten Benutzer und den durch den Verwaltungsteil für den Benutzer ausführbar gemachten Prozess korreliert und speichert.

22. Nahrungsmittelverarbeitungsvorrichtungs-Verwaltungssystem nach Anspruch 21, wobei:
der dritte Speicherteil konfiguriert ist, keine Änderungen oder Löschungen am korrelierten und gespeicherten Benutzer, der durch den Authentifizierungsteil authentifiziert wird, und am korrelierten und gespeicherten Prozess, der für den Benutzer durch den Verwaltungsteil ausführbar gemacht wird, anzunehmen.

23. Nahrungsmittelverarbeitungsvorrichtungs-Verwaltungssystem nach Anspruch 21 oder 22, wobei:
der dritte Speicherteil für jede der Nahrungsmittelverarbeitungsvorrichtungen den durch den Authentifizierungsteil authentifizierten Benutzer und den durch den Verwaltungsteil für den Benutzer ausführbar gemachten Prozess korreliert und speichert.

24. Nahrungsmittelverarbeitungsvorrichtungs-Verwaltungssystem nach einem der Ansprüche 16 bis 23, wobei:
der zweite Speicherteil die Benutzeridentifikationsinformationen und die Ausführbarkeitsinformationen korreliert und speichert.

25. Nahrungsmittelverarbeitungsvorrichtungs-Verwaltungsverfahren zum Verwalten mehrerer Nahrungsmittelverarbeitungsvorrichtungen, die ein Nahrungsmittel verarbeiten, wobei das Nahrungsmittelverarbeitungsvorrichtungs-Verwaltungsverfahren aufweist:
Durchführen eines Empfindlichkeitseinstellungsprozesses unter Verwendung einer Röntgenprüfvorrichtung (10) einer der Nahrungsmittelverarbeitungsvorrichtungen, um die Empfindlichkeit zur Feststellung der Annehmbarkeit des Nahrungsmittels einzustellen,
Erfassen biometrischer Informationen von Benutzern der Nahrungsmittelverarbeitungsvorrichtungen;
Korrelieren und Speichern, für jeden Benutzer, der biometrischen Informationen und der
Benutzeridentifikationsinformationen zum eindeutigen Identifizieren des Benutzers;
Speichern von Ausführbarkeitsinformationen, die sich auf einen Prozess beziehen, der für den Benutzer unter Verwendung der Nahrungsmittelverarbeitungsvorrichtung ausführbar ist;
Authentifizieren des Benutzers auf der Grundlage der erfassten biometrischen Informationen und der in Korrelation mit den Benutzeridentifikationsinformationen gespeicherten biometrischen Informationen; und
Ausführbarmachen des von der Nahrungsmittelverarbeitungsvorrichtung durchgeführten Prozesses für den authentifizierten Benutzer auf der Grundlage der gespeicherten Ausführbarkeitsinformationen;
**dadurch gekennzeichnet, dass**
die biometrische Informationen Informationen über die Iris des Benutzers sind, die in einem Bild des Auges des Benutzers enthalten ist,
nur dann, wenn der Authentifizierungsteil (63) den Benutzer authentifiziert, Steuern der Röntgenprüfvorrichtung (10) durch den Verwaltungsteil (64), so dass der authentifizierte Benutzer in der Lage ist, den von der Röntgenprüfvorrichtung (10) durchgeführten Empfindlichkeitseinstellungsprozess zu starten.

## Revendications

1. Dispositif de traitement de produit alimentaire prévu pour traiter un produit alimentaire dans une chaîne de production dudit produit alimentaire, ledit dispositif de traitement de produit alimentaire comprenant :
un dispositif d'inspection par rayons X (10),
un processeur (61) prévu pour exécuter, au moyen du dispositif d'inspection par rayons X (10), au moins un processus de réglage de sensibilité afin de déterminer l'acceptabilité du produit alimentaire ;
un dispositif d'imagerie prévu pour capturer une image de l'œil d'un utilisateur du dispositif de traitement de produit alimentaire ;
une unité d'authentification (63) prévue pour authentifier l'utilisateur sur la base de l'image de l'œil de l'utilisateur capturée par le dispositif d'imagerie ; et
une unité de gestion (64) prévue pour rendre le processus exécuté par le processeur exécutable pour l'utilisateur authentifié par l'unité d'authentification,
**caractérisé en ce que**
l'unité d'authentification est prévue pour authentifier l'utilisateur sur la base des informations de l'iris de l'utilisateur comprises dans l'image de l'œil de l'utilisateur,
uniquement si l'unité d'authentification (63) authentifie l'utilisateur, l'unité de gestion (64) est prévue pour commander le dispositif d'inspection par rayons X (10) de sorte que l'utilisateur authentifié puisse lancer le processus de réglage de sensibilité exécuté par le processeur (61).

2. Dispositif de traitement de produit alimentaire selon la revendication 1, où :
le dispositif de traitement de produit alimentaire comprend en outre une unité de détermination prévue pour déterminer si l'utilisateur est présent ou non à l'intérieur d'une zone prescrite ; et
l'unité de gestion est prévue pour mettre fin automatiquement à l'état exécutable du processus exécuté par le processeur sur la base d'un résultat de détermination provenant de l'unité de détermination.

3. Dispositif de traitement de produit alimentaire selon la revendication 2, où :
l'unité de détermination est prévue pour déterminer si l'utilisateur est présent ou non à l'intérieur de la zone prescrite à proximité du dispositif de traitement de produit alimentaire ; et
l'unité de gestion est prévue pour mettre automatiquement fin à l'état exécutable du processus exécuté par le processeur lorsque l'unité de détermination détermine que l'utilisateur n'est pas présent à l'intérieur de la zone prescrite.

4. Dispositif de traitement de produit alimentaire selon la revendication 3, où :
l'unité de détermination est prévue pour détecter si un indicateur détenu par l'utilisateur est présent à l'intérieur de la zone prescrite afin de déterminer si l'utilisateur est présent à l'intérieur de la zone prescrite.

5. Dispositif de traitement de produit alimentaire selon la revendication 4, où :
le dispositif de traitement de produit alimentaire comprend en outre une première unité de mémorisation mettant en corrélation et mémorisant l'utilisateur et l'indicateur ;
l'unité de détermination peut détecter une pluralité d'indicateurs distincts les uns des autres ; et
l'unité de détermination est prévue pour déterminer que l'utilisateur n'est pas présent dans la zone prescrite lorsqu'elle détecte que l'indicateur mémorisé dans la première unité de mémorisation et correspondant à l'utilisateur authentifié par l'unité d'authentification n'est pas présent dans la zone prescrite.

6. Dispositif de traitement de produit alimentaire selon l'une des revendications 1 à 5, où ledit dispositif de traitement de produit alimentaire comprend en outre :
une deuxième unité de mémorisation mettant en corrélation et mémorisant l'utilisateur et le processus rendu exécutable pour l'utilisateur par l'unité de gestion.

7. Dispositif de traitement de produit alimentaire selon la revendication 6, où :
la deuxième unité de mémorisation met en corrélation et mémorise
un niveau d'authentification faisant partie d'une pluralité de niveaux d'authentification et défini pour chaque utilisateur, et
le processus rendu exécutable par l'unité de gestion, ledit processus étant défini pour chaque niveau d'authentification.

8. Dispositif de traitement de produit alimentaire selon la revendication 7, où :
l'unité d'authentification est prévue pour authentifier en outre, sur la base d'informations autres qu'une image de l'œil de l'utilisateur, les utilisateurs pour lesquels le niveau d'authentification le plus élevé parmi la pluralité de niveaux d'authentification a été défini.

9. Dispositif de traitement de produit alimentaire selon l'une des revendications 1 à 8, où :
le processeur est prévu pour exécuter au moins deux types de processus, incluant un processus de premier type et un processus de deuxième type, sur le produit alimentaire ; et
l'unité de gestion est prévue pour rendre exécutable le processus de premier type exécuté par le processeur, uniquement pour l'utilisateur authentifié par l'unité d'authentification, et pour rendre le processus de deuxième type exécuté par le processeur exécutable pour tous les utilisateurs.

10. Dispositif de traitement de produit alimentaire selon l'une des revendications 1 à 9, où le dispositif de traitement de produit alimentaire comprend en outre :
une troisième unité de mémorisation mettant en corrélation et mémorisant l'utilisateur authentifié par l'unité d'authentification et le processus rendu exécutable pour l'utilisateur par l'unité de gestion.

11. Dispositif de traitement de produit alimentaire selon la revendication 10, où :
la troisième unité de mémorisation est prévue pour ne pas accepter de modifications ou de suppressions de l'utilisateur corrélé et mémorisé authentifié par l'unité d'authentification et du processus corrélé et mémorisé rendu exécutable pour l'utilisateur par l'unité de gestion.

12. Dispositif de traitement de produit alimentaire selon l'une des revendications 1 à 11, où le dispositif de traitement de produit alimentaire comprend en outre :
une quatrième unité de mémorisation mettant en corrélation et mémorisant l'image de l'œil de l'utilisateur capturée par le dispositif d'imagerie et les informations d'identification de l'utilisateur pour identifier l'utilisateur de manière unique.

13. Dispositif de traitement de produit alimentaire selon l'une des revendications 1 à 12, où :
le dispositif d'imagerie est prévu de telle sorte qu'au moins une position et une orientation du dispositif d'imagerie sont réglables en fonction de la position de l'œil de l'utilisateur.

14. Dispositif de traitement de produit alimentaire selon l'une des revendications 1 à 13, où :
lorsqu'un temps prescrit s'est écoulé après que l'utilisateur a été authentifié par l'unité d'authentification, l'unité de gestion rend inexécutable le processus exécuté par le processeur jusqu'à ce que l'utilisateur soit à nouveau authentifié par l'unité d'authentification.

15. Dispositif de traitement de produit alimentaire selon l'une des revendications 1 à 14, où :
le processeur est prévu pour exécuter le processus de détermination de présence/d'absence de substances étrangères comprises dans le produit alimentaire.

16. Système de gestion de dispositifs de traitement de produit alimentaire (200), prévu pour gérer une pluralité de dispositifs de traitement de produit alimentaire traitant un produit alimentaire dans une chaîne de fabrication dudit produit alimentaire, ledit système de gestion de dispositifs de traitement de produit alimentaire (200) comprenant :
les dispositifs de traitement de produit alimentaire qui pourvus chacun d'un dispositif d'inspection par rayons X (10), ledit dispositif d'inspection par rayons X (10) étant prévu pour exécuter un processus de réglage de sensibilité au moyen du dispositif d'inspection par rayons X (10), afin de régler la sensibilité pour déterminer l'acceptabilité du produit alimentaire,
une unité d'acquisition d'informations biométriques (60) prévue pour acquérir des informations biométriques sur les utilisateurs des dispositifs de traitement de produit alimentaire ;
une première unité de mémorisation (72) mettant en corrélation et mémorisant, pour chaque utilisateur, les informations biométriques et les informations d'identification de l'utilisateur afin d'identifier l'utilisateur de manière unique ;
une deuxième unité de mémorisation (71) mémorisant les informations d'exécutabilité relatives à un processus que les utilisateurs peuvent exécuter au moyen des dispositifs de traitement de produit alimentaire ;
une unité d'authentification (63) prévue pour authentifier les utilisateurs sur la base des informations biométriques acquises par l'unité d'acquisition d'informations biométriques et des informations biométriques mémorisées dans la première unité de mémorisation (72) ; et
une unité de gestion (64) prévue pour, sur la base des informations d'exécutabilité mémorisées dans la deuxième unité de mémorisation (71), rendre le processus exécuté par les dispositifs de traitement de produit alimentaire exécutable pour les utilisateurs authentifiés par l'unité d'authentification (63),
**caractérisé en ce que**
les informations biométriques sont des informations d'iris de l'utilisateur comprises dans l'image de l'œil de l'utilisateur,
uniquement si l'unité d'authentification (63) authentifie l'utilisateur, l'unité de gestion (64) est prévue pour commander le dispositif d'inspection par rayons X (10) de sorte que l'utilisateur authentifié puisse lancer le processus de réglage de sensibilité exécuté par le dispositif d'inspection par rayons X (10).

17. Système de gestion de dispositifs de traitement de produit alimentaire selon la revendication 16, comprenant en outre :
une pluralité de dispositifs de traitement de produit alimentaire.

18. Système de gestion de dispositifs de traitement de produit alimentaire selon la revendication 17, où :
les dispositifs de traitement de produit alimentaire sont prévus pour exécuter le processus de détermination de présence/d'absence de substances étrangères comprises dans le produit alimentaire.

19. Système de gestion de dispositifs de traitement de produit alimentaire selon la revendication 17 ou la revendication 18, où :
chacun des dispositifs de traitement de produit alimentaire comporte au moins une unité d'acquisition d'informations biométriques ; et
la première unité de mémorisation met en corrélation et mémorise les informations d'identification de l'utilisateur et les informations biométriques acquises par l'une quelconque des unités d'acquisition d'informations biométriques.

20. Système de gestion de dispositifs de traitement de produit alimentaire selon l'une des revendications 17 à 19, où : chaque dispositif de la pluralité de dispositifs de traitement de produit alimentaire est pourvu de la deuxième unité de mémorisation.

21. Système de gestion de dispositifs de traitement de produit alimentaire selon l'une des revendications 16 à 20, comprenant en outre :
une troisième unité de mémorisation mettant en corrélation et mémorisant l'utilisateur authentifié par l'unité d'authentification et le processus rendu exécutable pour l'utilisateur par l'unité de gestion.

22. Système de gestion de dispositifs de traitement de produit alimentaire selon la revendication 21, où :
la troisième unité de mémorisation est prévue pour ne pas accepter de modifications ou de suppressions de l'utilisateur corrélé et mémorisé authentifié par l'unité d'authentification et du processus corrélé et mémorisé rendu exécutable pour l'utilisateur par l'unité de gestion.

23. Système de gestion de dispositifs de traitement de produit alimentaire selon la revendication 21 ou la revendication 22, où :
la troisième unité de mémorisation met en corrélation et mémorise, pour chacun des dispositifs de traitement de produit alimentaire, l'utilisateur authentifié par l'unité d'authentification et le processus rendu exécutable pour l'utilisateur par l'unité de gestion.

24. Système de gestion de dispositifs de traitement de produit alimentaire selon l'une des revendications 16 à 23, où :
la deuxième unité de mémorisation met en corrélation et mémorise les informations d'identification de l'utilisateur et les informations d'exécutabilité.

25. Procédé de gestion de dispositifs de traitement de produit alimentaire, pour la gestion d'une pluralité de dispositifs de traitement de produit alimentaire traitant un produit alimentaire, ledit procédé de gestion de dispositifs de traitement de produit alimentaire comprenant :
l'exécution d'un processus de réglage de sensibilité au moyen d'un dispositif d'inspection par rayons X (10) de l'un des dispositifs de traitement de produit alimentaire, afin de régler la sensibilité pour déterminer l'acceptabilité du produit alimentaire,
l'acquisition d'informations biométriques sur les utilisateurs des dispositifs de traitement des denrées alimentaires ;
la corrélation et la mémorisation, pour chaque utilisateur,
des informations biométriques et des informations d'identification de l'utilisateur afin d'identifier l'utilisateur de manière unique ;
la mémorisation des informations relatives à l'exécution d'un processus exécutable par l'utilisateur au moyen du dispositif de traitement de produit alimentaire ;
l'authentification de l'utilisateur sur la base des informations biométriques acquises et des informations biométriques mémorisées en corrélation avec les informations d'identification de l'utilisateur ; et
la disposition de l'exécutabilité du processus exécuté par le dispositif de traitement de produit alimentaire pour l'utilisateur authentifié sur la base des informations d'exécutabilité mémorisées ;
**caractérisé en ce que**
les informations biométriques sont des informations sur l'iris de l'utilisateur comprises dans une image de l'œil de l'utilisateur,
uniquement si l'unité d'authentification (63) authentifie l'utilisateur, le dispositif d'inspection par rayons X (10) est commandé par l'unité de gestion (64) afin que l'utilisateur authentifié puisse lancer le processus de réglage de sensibilité exécuté par le dispositif d'inspection par rayons X (10).
